# EUROPEAN PATENT SPECIFICATION

(11) **EP 3 116 439 B1**
(45) Date of publication and mention of the grant of the patent: **07.02.2018**
(21) Application number: 15711020.6
(22) Date of filing: 10.03.2015
(51) Int. Cl.: A61F 2/07, A61F 2/954, A61F 2/962, A61F 2/06

(54) **INFLATABLE OCCLUSION WIRE-BALLOON FOR AORTIC APPLICATIONS**
AUFBLASBARER OKKLUSIONSDRAHTBALLON FÜR ANWENDUNGEN IN DER AORTA
BALLONNET DE FIL D'OCCLUSION GONFLABLE POUR APPLICATIONS AORTIQUES

(30) Priority: 10.03.2014 US 201461950461 P
(43) Date of publication of application: 18.01.2017
(73) Proprietor: TriVascular, Inc., Santa Rosa, CA 95403 (US)
(72) Inventor: CHOBOTOV, Michael, V., Santa Rosa, CA 95403 (US)
(74) Representative: EP&C
(86) International application number: PCT/US2015/019626
(87) International publication number: WO 2015/138402

(56) References cited:
- WO-A2-2005/039442
- US-A- 5 571 172
- US-A1- 2005 085 842
- US-A1- 2013 338 760

## Description

### CROSS-REFERENCE TO RELATED APPLICATIONS:

This application claims the benefit of U.S. Provisional Application No. 61/950,461, filed March 10, 2014.

### FIELD OF THE INVENTION:

The present invention is related to an endovascular delivery system for an endovascular prosthesis. More particularly, the present invention is related to an endovascular delivery system having an inflatable occlusion wire-balloon for aortic applications where rupture of an aneurysm may be a concern for a patient's health.

### BACKGROUND OF THE INVENTION:

An aneurysm is a medical condition indicated generally by an expansion and weakening of the wall of an artery of a patient. Aneurysms can develop at various sites within a patient's body. Thoracic aortic aneurysms (TAAs) or abdominal aortic aneurysms (AAAs) are manifested by an expansion and weakening of the aorta which is a serious and life threatening condition for which intervention is generally indicated. Existing methods of treating aneurysms include invasive surgical procedures with graft replacement of the affected vessel or body lumen or reinforcement of the vessel with a graft.

Surgical procedures to treat aortic aneurysms can have relatively high morbidity and mortality rates due to the risk factors inherent to surgical repair of this disease as well as long hospital stays and painful recoveries. This is especially true for surgical repair of TAAs, which is generally regarded as involving higher risk and more difficulty when compared to surgical repair of AAAs. An example of a surgical procedure involving repair of a AAA is described in a book titled Surgical Treatment of Aortic Aneurysms by Denton A. Cooley, M.D., published in 1986 by W.B. Saunders Company.

Due to the inherent risks and complexities of surgical repair of aortic aneurysms, endovascular repair has become a widely-used alternative therapy, most notably in treating AAAs. Early work in this field is exemplified by Lawrence, Jr. et al. in "Percutaneous Endovascular Graft Experimental Evaluation", Radiology (May 1987) and by Mirich et al. in "Percutaneously Placed Endovascular Grafts for Aortic Aneurysms: Feasibility Study," Radiology (March 1989). Commercially available endoprostheses for the endovascular treatment of AAAs include the Endurant™ and Talent™ Abdominal Stent Grafts sold by Medtronic, Inc. of Minneapolis, MN; the Zenith Flex® AAA Endovascular Graft and the Zenith TX2® TAA Endovascular Graft, both sold by Cook Medical, Inc. of Bloomington, IN; the AFX™ Endovascular AAA system sold by Endologix, Inc. of Irvine, CA; and the Gore® Excluder® AAA Endoprosthesis sold by W.L. Gore & Associates, Inc. of Flagstaff, AZ. A commercially available stent graft for the treatment of TAAs is the Gore® TAG® Thoracic Endoprosthesis sold by W.L. Gore & Associates, Inc. of Flagstaff, AZ.

It is believed that not an insignificant number of aortic aneurysms, such as up to about seven percent, may involve priority medical procedures, such as endovascular repair (EVAR). Such priority medical procedures are concerned with potential rupture of an aneurysm. Use of occlusion balloons to avoid blood loss in patients with potential rupture of aneurysms has been proposed. See for example, Philipsen, Tine E. et al, "The Use of Rapid Endovascular Balloon Occlusion in Unstable Patients With Ruptured Abdominal Aortic Aneurysm", Innovations, Volume 4, Number 2, March/April 2009, pages 74-79; Mehta, Manish, "Compliant Occlusion Balloons - Use of Compliant Occlusion Balloons During EVAR for AAA Rupture", Insert to Endovascular Today, November 2008, pages 29-31. Such proposed uses involve separate occlusion balloon catheters and separate EVAR delivery device catheters. What have been needed are stent graft systems, delivery systems and methods that are adaptable to a wide range of patient anatomies and that can be safely and reliably deployed using a flexible low profile system having integrated occlusion balloon capability.

In US 2013/338760 A1, an endovascular delivery system is disclosed, which included a bifurcated and inflatable prosthesis including a main tubular body having an open end and opposed ipsilateral and contralateral legs defining a graft wall therein between.

In US 5,571,172 A. a method and apparatus are disclosed for reconstructing target tissue by grafting, using a stabilizing balloon cannula having an endoscope for visualizing the placement of the graft during the procedure.

### SUMMARY OF THE INVENTION:

In one aspect of the present invention an endovascular delivery system is provided. The endovascular delivery system may comprise: a prosthesis comprising a main tubular body having an open proximal end and opposed open ipsilateral and contralateral legs; an elongate outer tubular sheath having an open lumen and opposed proximal and distal ends with a medial portion therein between; an elongate inner tubular member slidably disposed within the open lumen of the outer tubular sheath; wherein the distal end of the outer tubular sheath being slidably disposed past and beyond the distal end of the inner tubular member to define a prosthesis delivery state and slidably retractable to the medial portion of the inner tubular member to define a prosthesis unsheathed state; an elongate guidewire slidably disposed within the inner tubular member and extending from the handle assembly, through the ipsilateral leg of the prosthesis and through the main tubular body of the prosthesis and extending past the open of the main tubular body in the prosthesis delivery state, said elongate guidewire comprising a hollow portion such that the inflatable occlusion balloon is in fluid communication with a balloon inflation material; and an inflatable occlusion balloon disposed on a portion of the elongate guidewire extending past the open end of the main tubular body. The main tubular body may comprise an inflatable cuff disposed near the open proximal end.

The elongate guidewire may comprise a hollow portion such that the inflatable occlusion balloon is in fluid communication with a balloon inflation material. Further, the portion of the elongate guidewire over which the occlusion balloon is disposed may haves porosity, such as a hole or a plurality of holes, for ingress and egress of the balloon inflation material. Moreover, the balloon inflation material may comprise saline and contrast material.

The elongate guidewire may comprise a metallic material, a polymeric material or a combination thereof.

The endovascular delivery system may further comprise a removable mandrel disposed at least partially within hollow portion of the elongate guidewire for supporting elongate guidewire prior to inflation of the occlusion balloon. Moreover, the endovascular delivery system may further comprise a seal disposed on a portion of the elongate guidewire disposed within the handle assembly.

The endovascular delivery system may further comprise a first radiopaque marker disposed on a portion of the elongate guidewire just past the occlusion balloon and/or a second radiopaque marker disposed on a portion of the elongate guidewire just prior the occlusion balloon.

In another aspect of the present disclosure, an endovascular delivery system may comprise a prosthesis comprising a main tubular body having an open end and opposed open end; an elongate outer tubular sheath having an open lumen and opposed proximal and distal ends with a medial portion therein between; an elongate guidewire slidably disposed within the inner tubular member and extending from the handle assembly, through the ipsilateral leg of the prosthesis and through the main tubular body of the prosthesis and extending past the open of the main tubular body in the prosthesis delivery state, said elongate guidewire comprising a hollow portion such that the inflatable occlusion balloon is in fluid communication with a balloon inflation material; an elongate guidewire slidably disposed within the inner tubular member and extending from the handle assembly through the main tubular body of the prosthesis and extending past the open of the main tubular body in the prosthesis delivery state; and an inflatable occlusion balloon disposed on a portion of the elongate guidewire extending past the open end of the main tubular body.

In another aspect of the present disclosure, a method for delivering a bifurcated prosthesis may comprise providing the endovascular delivery system of the present invention; advancing the endovascular delivery system through a first branched artery and into an aneurysm in a main artery; retracting the outer sheath so that the proximal end of the main tubular body of the prosthesis is disposed beyond the aneurysm and so that the ipsilateral and contralateral legs are disposed within the aneurysm; inflating the occlusion balloon with an inflation material in the main artery beyond the aneurysm to provide a seal against blood flow thereat; deploying the prosthesis; inflating the inflatable cuff of the main tubular body to provide a seal against blood in the main artery beyond the aneurysm; and deflating the occlusion balloon.

The method may further comprise: deploying a contralateral graft extension having opposed proximal and distal open ends contained within a catheter so that the proximal end of the contralateral graft extension is disposed within a portion of the contralateral leg of the main tubular body of the prosthesis and so that the distal end of the contralateral graft extension is disposed distally of the aneurysm and within a portion of a second branched artery. Moreover, the method may further comprise: deploying a ipsilateral graft extension having opposed proximal and distal open ends contained within a second catheter so that the proximal end of the ipsilateral graft extension is disposed within a portion of the ipsilateral leg of the main tubular body of the prosthesis and so that the distal end of the ipsilateral graft extension is disposed distally of the aneurysm and within a portion of the first branched artery.

In another aspect of the present disclosure, an assembly for rapid endovascular balloon occlusion may comprise: a guidewire having a hollow lumen portion, a proximal portion, a distal portion and a balloon mounting portion therein between; a non-compliant or semi-compliant occlusion balloon securably disposed on the balloon mounting portion of the guidewire and in fluid communication with the hollow lumen portion; and a catheter having a sheath having the non-compliant or semi-compliant balloon and portions of the guidewire slidably disposed therein. The assembly may further comprise: a syringe having inflation material for inflating the non-compliant or semi-compliant balloon; and a luer fitting for providing fluid communication of the inflation material to the hollow lumen portion of the guidewire. Further, the guidewire may have an outer diameter of about 0.035 inches (0.9 mm) and a hollow lumen diameter of about 0.015 inches (0.38 mm) to about 0.030 inches (0.76 mm).

In another aspect of the present disclosure, a method for rapid endovascular balloon occlusion may comprise: providing a guidewire having a hollow lumen portion, a proximal portion, a distal portion and a balloon mounting portion therein between; providing a non-compliant or semi-compliant occlusion balloon securably disposed on the balloon mounting portion of the guidewire and in fluid communication with the hollow lumen portion; providing a catheter having a sheath having the non-compliant or semi-compliant balloon and portions of the thin guidewire slidably disposed therein; providing a syringe having inflation material for inflating the non-compliant or semi-compliant balloon; and a luer fitting for providing fluid communication of the inflation material to the hollow lumen portion of the guidewire; delivering the catheter to a desired bodily location; withdrawing the sheath of the catheter to expose the occlusion balloon; and inflating the occlusion balloon with the inflation material. The occlusion balloon may be inflated with the inflation material within about 15 to about 30 seconds. The guidewire may have an outer diameter of about 0.035 inches (0.9 mm) and a hollow lumen diameter of about 0.015 inches (0.38 mm) to about 0.030 inches (0.76 mm).

These and other features and advantages of the present invention will become apparent from the following detailed description of illustrative embodiments thereof, which is to be read in connection with the accompanying drawings. Corresponding reference element numbers or characters indicate corresponding parts throughout the several views of the drawings.

### BRIEF DESCRIPTION OF THE DRAWINGS:

FIG. 1 depicts an initial deployment state of an embodiment of the endovascular delivery system of the present invention within a patient's vasculature.
FIG. 2 depicts a deployment state of an embodiment of the endovascular delivery system, including an unexpanded occlusion balloon, of the present invention within a patient's vasculature after withdrawal of an outer sheath.
FIG. 3 depicts a deployment state of an embodiment of the endovascular delivery system of the present invention within a patient's vasculature after withdrawal of an outer sheath and after inflation of the occlusion balloon.
FIG. 4 depicts a further deployment state of an embodiment of the endovascular delivery system of the present invention within a patient's vasculature after an initial and partial stent deployment while the occlusion balloon is in its inflated state.
FIG. 5 depicts a further deployment state of an embodiment of the endovascular delivery system of the present invention within a patient's vasculature after further deployment of a stent while the occlusion balloon remains in its inflated state.
FIG. 6 depicts a deployment state of an embodiment of the endovascular delivery system of the present invention within a patient's vasculature after further deployment of a stent (as shown in FIG. 5) after at least partial deflation of the occlusion balloon.
FIG. 7 depicts a deployed bifurcated endovascular prosthesis with graft leg extensions.
FIG. 8 is a side elevational view of an embodiment of an endovascular delivery system of the present invention.
FIG. 9 is a side elevational and partial cutaway view of the distal portion of an embodiment of the endovascular delivery system of the present invention.
FIG. 10 is a partial perspective and partial cutaway view of the distal portion of an embodiment of the endovascular delivery system of the present invention.
FIG. 11 is a perspective view of the inner tubular member or hypotube of an embodiment of the endovascular delivery system of the present invention.
FIG. 12 is a partial perspective and cutaway view of a distal portion of an embodiment of the endovascular delivery system of the present invention showing a distal end portion of the hypotube.
FIG. 13 is a side elevational view of an embodiment of an occlusion balloon of the present invention in an uninflated state on a delivery guidewire.
FIG. 14 is an exploded view of a portion of the occlusion balloon of FIG. 13 denoted by an area A in FIG. 13.
FIG. 15 is a side elevational view of the occlusion balloon of an embodiment of the present invention in an inflated state on a delivery guidewire.
FIG. 16 is a perspective view of a portion of the hollow guidewire according an embodiment of to an embodiment of the present invention taken along the B-B axis of FIG. 15.
FIG. 17 is a perspective view of a portion of a hollow polymeric guidewire having a reinforcing member according to an embodiment of the present invention.
FIG. 18 is a cross-sectional of the hollow guidewire taken along the C-C axis of FIG. 17.
FIG. 19A is a side elevational view of an embodiment of a guidewire and balloon assembly of the present invention.
FIG. 19B is a side elevational view of an embodiment of a delivery catheter having the guidewire and balloon assembly of FIG. 19A of the present invention.
FIG. 20 is a partial side elevational view of an embodiment of a hollow guidewire having a slidable elongate mandrel within the open lumen of the guidewire according to an embodiment of the present invention.
FIG. 21 is side elevational, partial perspective view of a luer-type fitting useful for inflating the occlusion balloon according embodiments of to the present invention.
FIG. 22 is a side elevational view of a syringe useful for inflating and/or deflating embodiments of the occlusion balloon of the present invention.

### DETAILED DESCRIPTION OF THE INVENTION:

Embodiments of the invention are directed generally to methods and devices for treatment of fluid flow vessels with the body of a patient, including EVAR methods and devices. Treatment of blood vessels is specifically indicated for some embodiments, and, more specifically, treatment of aneurysms, such as abdominal aortic aneurysms. With regard to endovascular prosthesis or stent/stent-graft embodiments discussed herein and components or portions thereof, the term "proximal" refers to a location towards a patient's heart and the term "distal" refers to a location away from the patient's heart. With regard to delivery system catheters and components thereof discussed herein, the term "distal" refers to a location that is disposed away from an operator who is using the catheter and the term "proximal" refers to a location towards the operator.

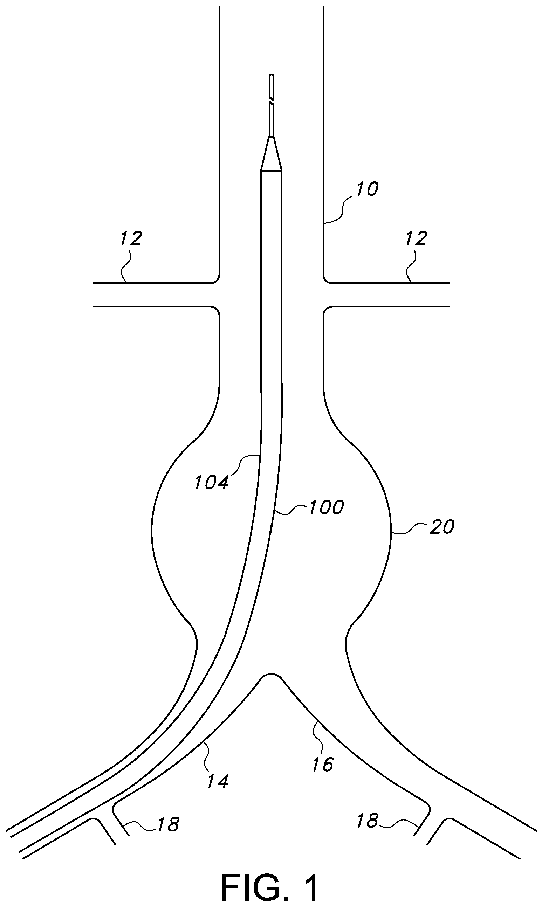

FIG. 1 illustrates an embodiment of a deployment sequence of an embodiment of an endovascular prosthesis (not shown), such as a modular stent-graft assembly. For endovascular methods, access to a patient's vasculature may be achieved by performing an arteriotomy or cut down to the patient's femoral artery or by other common techniques, such as the percutaneous Seldinger technique. For such techniques, a delivery sheath (not shown) may be placed in communication with the interior of the patient's vessel such as the femoral artery with the use of a dilator and guidewire assembly. Once the delivery sheath is positioned, access to the patient's vasculature may be achieved through the delivery sheath which may optionally be sealed by a hemostasis valve or other suitable mechanism. For some procedures, it may be necessary to obtain access via a delivery sheath or other suitable means to both femoral arteries of a patient with the delivery sheaths directed upstream towards the patient's aorta. In some applications a delivery sheath may not be needed and the delivery catheter of the present invention may be directly inserted into the patient's access vessel by either arteriotomy or percutaneous puncture. Once any delivery sheath or sheaths, if used, have been properly positioned, an endovascular delivery catheter or system, typically containing an endovascular prosthesis such as an inflatable stent-graft, may then be advanced over a guidewire through the delivery sheath and into the patient's vasculature.

While an embodiment of the present invention is described hereinafter in relation to a particular inflatable stent-graft, the present invention may be used with any suitable endovascular prosthesis for use within a patient's vasculature, including branched and non-branched bodily lumens. The endovascular prosthesis may have inflatable cuffs and/or channels for sealing against vessel walls, such as non-aneurysmal vessel walls. Alternatively or in addition to, the endovascular prosthesis may include inflatable components to seal or strengthen aneurysmal walls or to occupy aneurysmal sac areas. Use of a non-inflatable endovascular prosthesis, either alone or in combination with other inflatable or non-inflatable endovascular prostheses may be suitably used with the embodiments of the present invention. Further, suitable endovascular prostheses may include stent-grafts, grafts without stents; grafts with anchoring, typically expandable members either directly associated with the grafts or indirectly associated with grafts through the use of connecting members, such as struts, tethers, sutures. Further, useful endovascular prostheses include modular prostheses and uni-body prostheses. Thus, the embodiments of the delivery system according to the present invention may be used with a variety of endovascular prostheses.

Further, while embodiments of the present invention are described for use in the treatment of abdominal aortic aneurysms (AAAs), the present invention is not so limited. For example, embodiments of the present invention may be used in the treatment of thoracic aortic aneurysms (TAAs), other aortic aneurysms, arteriovenous aneurysms, artherosclerotic aneurysms, compound aneurysms, dissecting aneurysms, fusiform aneurysms, mycotic aneurysms, sacculated aneurysms. Further, embodiments of the present invention may be used in the treatment of bodily lumens at, near or remote from branched lumens.

FIG. 1 depicts the initial placement of the endovascular delivery system 100 of the present invention within a patient's vasculature. The endovascular delivery system 100 may be advanced along a guidewire 102 proximally upstream of blood flow into the vasculature of the patient including iliac arteries 14, 16 and aorta 10 shown in FIG. 1. While the iliac arties 14, 16 may be medically described as the right and left common iliac arteries, respectively, as used herein iliac artery 14 is described as an ipsilateral iliac artery and iliac artery 16 is described as a contralateral iliac artery. The flow of the patient's blood (not shown) is in a general downward direction in FIG. 1. Other vessels of the patient's vasculature shown in FIG. 1 include renal arteries 12 and hypogastric arteries 18.

The endovascular delivery system 100 may be advanced into the aorta 10 of the patient until the endovascular prosthesis (not shown) is disposed substantially adjacent an aortic aneurysm 20 or other vascular defect to be treated. The portion of the endovascular delivery system 100 that is advanced through bodily lumens is in some embodiments a low profile delivery system; for example, having an overall outer diameter of less than 14 French. Other diameters are also useful, such as less than 12 French, less than 10 French, or any sizes from 10 to 14 French or greater. The diameter (D) of a round catheter in millimeters can be determined by dividing the French (Fr) size by 3, i.e., D (mm) = Fr / 3. Once the endovascular delivery system 100 is so positioned, an outer sheath 104 of the endovascular delivery system 100 may be retracted distally so as to expose the prosthesis (not shown) which has been compressed and compacted to fit within the inner lumen of the outer sheath 104 of the endovascular delivery system 100. The present invention, however, is not limited to delivery systems requiring the retraction of an outer sheath for exposing a prosthesis and other techniques may suitably be used, such as, splitable sheaths, compressing coils, sutures or threads.

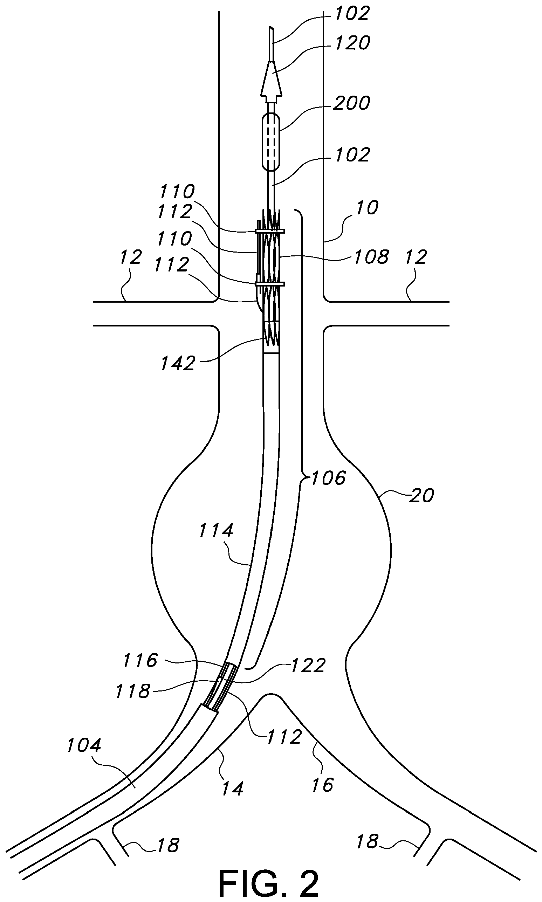

As depicted in FIG. 2, once the endovascular delivery system 100 is so positioned, the outer sheath 104 of the endovascular delivery system 100 may be retracted distally so as to expose the endovascular prosthesis 106 which has been compressed and compacted to fit within the inner lumen of the outer sheath 104 of the endovascular delivery system 100. The outer sheath 104 may be formed any suitable biocompatible material. In some embodiments, the biocompatible material may be a biocompatible polymer. Examples of suitable biocompatible polymers may include polyolefins such as polyethylene (PE), high density polyethylene (HDPE) and polypropylene (PP), polyolefin copolymers and terpolymers, polytetrafluoroethylene (PTFE), polyethylene terephthalate (PET), polyesters, polyamides, polyurethanes, polyurethaneureas, polypropylene and, polycarbonates, polyvinyl acetate, thermoplastic elastomers including polyether-polyester block copolymers and polyamide/polyether/polyesters elastomers, polyvinyl chloride, polystyrene, polyacrylate, polymethacrylate, polyacrylonitrile, polyacrylamide, silicone resins, combinations and copolymers thereof. In some embodiments, the biocompatible polymers include polypropylene (PP), polytetrafluoroethylene (PTFE), polyethylene terephthalate (PET), high density polyethylene (HDPE), combinations and copolymers thereof. The outer sheath 104 may optionally include a suitable biocompatible coating(s). Non-limiting examples of suitable coatings include polytetrafluoroethylene, silicone, hydrophilic materials, hydrogels. Useful hydrophilic coating materials may include alkylene glycols, alkoxy polyalkylene glycols such as methoxypolyethylene oxide, polyoxyalkylene glycols such as polyethylene oxide, polyethylene oxide/polypropylene oxide copolymers, polyalkylene oxide-modified polydimethylsiloxanes, polyphosphazenes, poly(2-ethyl-2-oxazoline), homopolymers and copolymers of (meth) acrylic acid, poly(acrylic acid), copolymers of maleic anhydride including copolymers of methylvinyl ether and maleic acid, pyrrolidones including poly(vinylpyrrolidone) homopolymers and copolymers of vinyl pyrrolidone, poly(vinylsulfonic acid), acryl amides including poly(N-alkylacrylamide), poly(vinyl alcohol), poly(ethyleneimine), polyamides, poly(carboxylic acids), methyl cellulose, carboxymethylcellulose, hydroxypropyl cellulose, polyvinylsulfonic acid, water soluble nylons, heparin, dextran, modified dextran, hydroxylated chitin, chondroitin sulphate, lecithin, hyaluranon, combinations and copolymers thereof. Non-limiting examples of suitable hydrogel coatings include polyethylene oxide and its copolymers, polyvinylpyrrolidone and its derivatives; hydroxyethylacrylates or hydroxyethyl(meth)acrylates; polyacrylic acids; polyacrylamides; polyethylene maleic anhydride, combinations and copolymers thereof. In some embodiments, the outer sheath 104 may be made of polymeric materials, e.g., polyimides, polyester elastomers (Hytrel®), or polyether block amides (Pebax®), polytetrafluoroethylene, and other thermoplastics and polymers. The outside diameter of the outer sheath 104 may range from about 0.1 inches (2.5 mm) to about 0.4 inches (10.2 mm). The wall thickness of the outer sheath 104 may range from about 0.002 inches (0.05 mm) to about 0.015 inches (0.38 mm). The outer sheath 104 may also include an outer hydrophilic coating. Further, the outer sheath 104 may include an internal braided or otherwise reinforced portion of metallic, polymeric, or other material- filaments or components, including combinations thereof.

In addition to being radially compressed when disposed within an inner lumen of the outer sheath 104 of the endovascular delivery system 100, embodiments of endovascular prostheses useful with the present invention may include a proximal stent 108 that may be radially restrained by high strength flexible belts 110 in order to maintain a small profile and avoid engagement of the proximal stent 108 with a body lumen wall until deployment of the proximal stent 108 is initiated. The use of such belts 110 is an example and other suitable restraining members may suitable be used. The belts 110 can be made from any high strength, resilient material that can accommodate the tensile requirements of the belt members and remain flexible after being set in a constraining configuration. Typically, belts 110 are made from solid ribbon or wire of a shape memory alloy such as nickel titanium, although other metallic or polymeric materials are possible. Belts 110 may also be made of braided metal filaments or braided or solid filaments of high strength synthetic fibers such as Dacron®, Spectra. An outside transverse cross section of the belts 110 may range from about 0.002 inches (0.05 mm) to about 0.012 inches (0.30 mm), specifically, about 0.004 inches (0.10 mm) to about 0.007 inches (0.18 mm). The cross sections of belts 110 may generally take on any shape, including rectangular (in the case of a ribbon), circular, elliptical, square. The ends of the belts 110 may be secured by one or more stent release wires or elongate rods 112 which extend through looped ends (not shown) of the belts 110. The stent release wires or elongate rods 112 may be disposed generally within the prosthesis 106 during delivery of the system 100 to the desired bodily location. For example, the stent release wires or elongate rods 112 may enter and exit the guidewire lumen 122 or other delivery system lumen as desired to affect controlled release of the stent 108, including if desired controlled and staged release of the stent 108. Once the outer sheath 104 of the endovascular delivery system 100 has been retracted, the endovascular delivery system 100 and the endovascular prosthesis 106 may be carefully positioned in an axial direction such that the proximal stent 108 is disposed substantially even with the renal arteries. Further details, including methods, catheters and systems, for deployment of endovascular prostheses are disclosed in commonly owned U.S. Patent Nos. 6,761,733 and 6,733,521 and commonly owned U.S. Patent Application Publication Nos. 2006/0009833 and 2009/0099649.

In some embodiments, the endovascular prosthesis 106 may include inflatable or non-inflatable unibody and/or bifurcated grafts, such as an inflatable bifurcated graft 114. The inflatable graft may be, for example, a bifurcated graft having a main graft body 124 (that may also be referred to as an "aortic body"), an ipsilateral graft leg 126 and a contralateral graft leg 128. The inflatable graft 114 may further include a fill port 116 in fluid communication with an inflation tube 118 of the endovascular delivery system 100 for providing an inflation medium (not shown). Examples of useful inflation medium may be found in U.S. Patent No. 8,535,705 and commonly owned U.S. Patent Application Publication Nos. 2011/0196060 and 2005/0158272. The distal portion of the endovascular delivery system 100 may optionally include a nosecone 120 which provides an atraumatic distal portion of the endovascular delivery system 100. The guidewire 102 is slidably disposed within a guidewire lumen 122 of the endovascular delivery system 100.

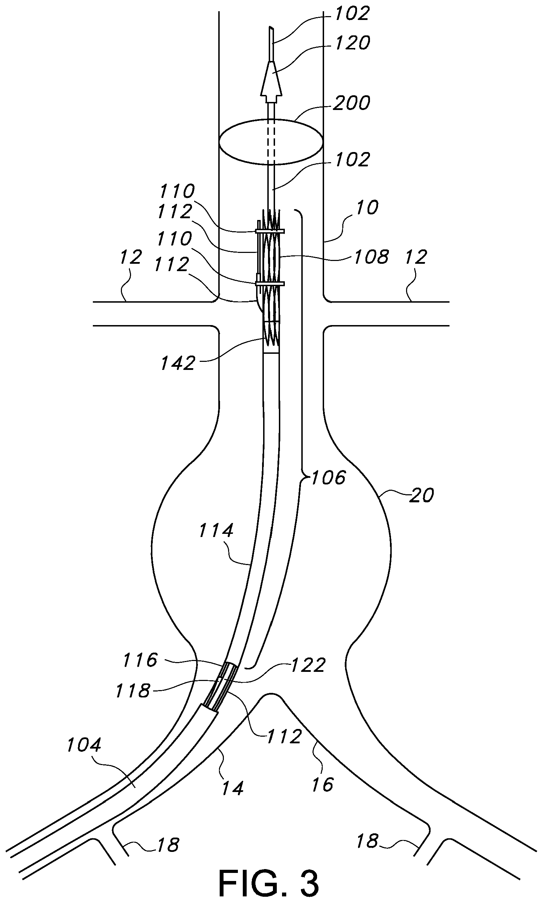

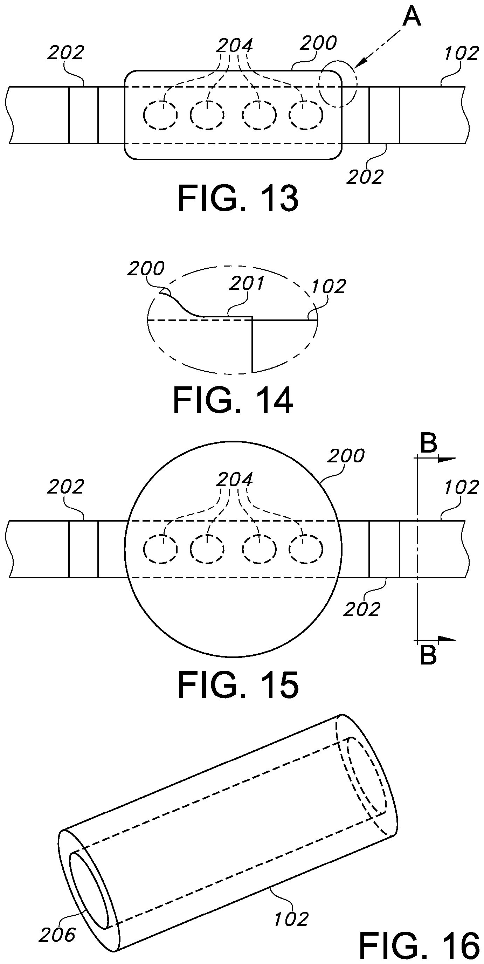

The guidewire 102 may include an occlusion balloon 200, which is depicted in its uninflated state or delivery state. As depicted in in FIG. 3, the occlusion balloon 200 may be inflated to occlude a vessel including a blood vessel, such as an aorta 10. While the outer sheath 104 is depicted in FIGS. 2 and 3 as being withdrawn to expose the endovascular prosthesis 106 when is the occlusion balloon 200 is exposed and/or inflated, the present invention is not so limited. The outer sheath 104 may be withdrawn to expose the occlusion balloon 200, but still cover the endovascular prosthesis 106, as the occlusion balloon 200 is to be inflated. Further, if controlling potential blood loss is urgent or critical, inflation of the occlusion balloon 200 prior to unsheathing of the delivery system 100 may be warranted. In such a case, the delivery system 100 may, for example, contain an unsheathed occlusion balloon 200 for rapid inflation protocol of the occlusion balloon 200, if required in such an urgent medical condition. Additional details for embodiments of the occlusion balloon 200 are described below in conjunction with FIGS. 13-22.

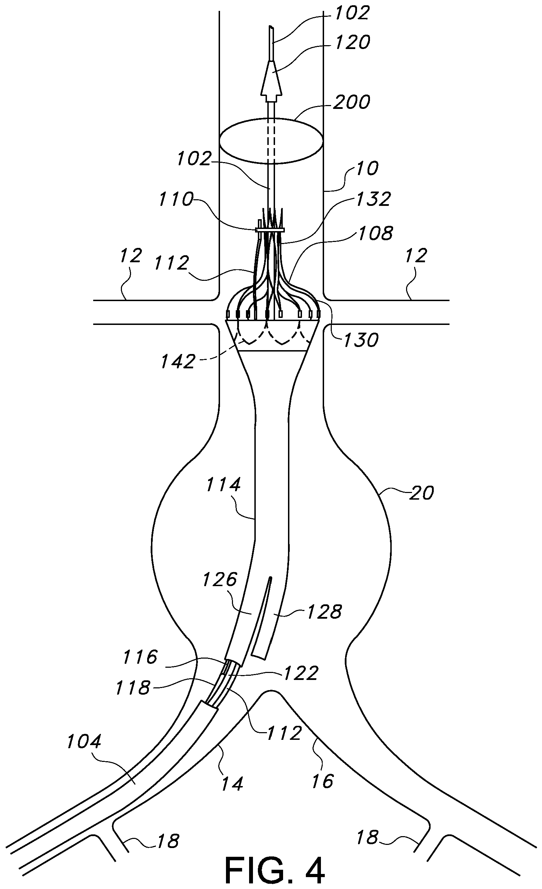

As depicted in FIG. 4, with the occlusion balloon 200 inflated, deployment of the proximal stent 108 may begin with deployment of the distal portion 130 of stent 108 by retracting the stent release wire or rod 112 that couples ends of belt 110 restraining the distal portion 130 of the stent 108. The distal portion 130 of stent 108 may be disposed to the main graft body 124 via a connector ring 142. The stent 108 and/or the connector ring 142 may be made from or include any biocompatible material, including metallic materials, such as nitinol (nickel titanium), cobalt-based alloy such as Elgiloy, platinum, gold, stainless steel, titanium, tantalum, niobium, and combinations thereof. The present invention, however, is not limited to the use of such a connector ring 142 and other shaped connectors for securing the distal portion 130 of the stent 108 at or near the end of the main graft body 124 may suitably be used. Additional axial positioning typically may be carried out even after deploying the distal portion 130 of the stent 108 as the distal portion 130 may provide only partial outward radial contact or frictional force on the inner lumen of the patient's vessel or aorta 10 until the proximal portion 132 of the stent 108 is deployed. Once the belt 110 constraining the proximal portion 132 of the stent 108 has been released, the proximal portion 132 of the stent 108 self-expands in an outward radial direction until an outside surface of the proximal portion 132 of the stent 108 makes contact with and engages an inner surface of the patient's vessel 10.

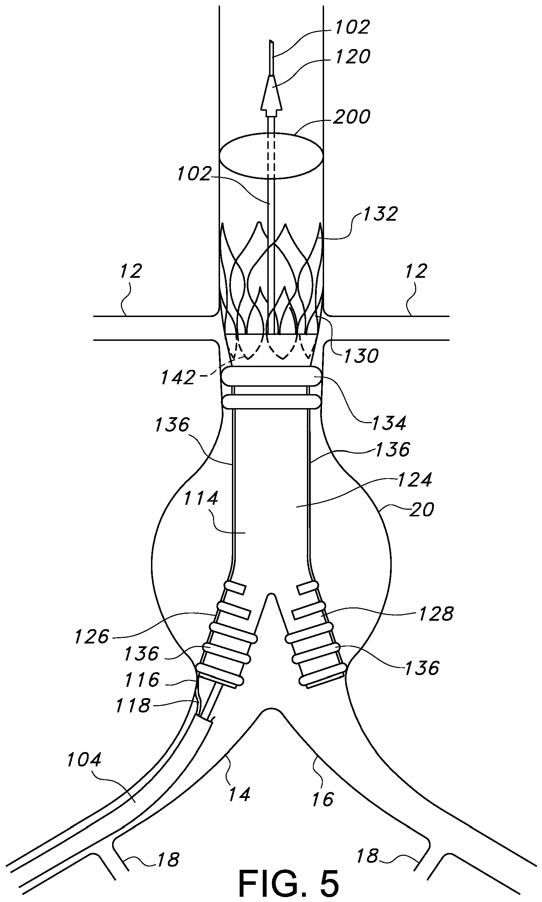

As depicted in FIG. 5, after the distal portion 130 of the stent 108 has been deployed, the proximal portion 132 of the stent 108 may then be deployed by retracting the wire 112 that couples the ends of the belt 110 restraining the proximal portion 132 of the stent 108. As the proximal portion 132 of the stent 108 self-expands in an outward radial direction, an outside surface of the proximal portion 132 of the stent 108 eventually makes contact with the inside surface of the patient's aorta 10. For embodiments that include tissue engaging members, e.g., anchors or barbs (not shown) on the proximal portion 132 of the stent 108, such barbs may be oriented such that upon stent expansion the barbs move in a general outward direction so as to make contact and engage the inner surface tissue of the patient's vessel or aorta 10, which further secures the proximal stent 108 to the patient's vessel 10.

Once the proximal stent 108 has been partially or fully deployed, the proximal inflatable cuff 134 of inflatable versions of prostheses that may be used in the present invention may then be filled through the inflation port 116 with inflation material injected through an inflation tube 118 of the endovascular delivery system 100 which may serve to seal an outside surface of the inflatable cuff 134 to the inside surface of the vessel 10. The remaining network of inflatable channels 136 may also be filled with pressurized inflation material at the same time which provides a more rigid frame like structure to the inflatable graft 114. For some embodiments, the inflation material may be a biocompatible, curable or hardenable material that may cured or hardened once the network of inflatable channels 136 are filled to a desired level of material or pressure within the network or after passage of a predetermined period of time. Some embodiments may also employ radiopaque inflation material to facilitate monitoring of the fill process and subsequent engagement of graft extensions (not shown). The material may be cured by any of the suitable methods discussed herein including time lapse, heat application, application of electromagnetic energy, ultrasonic energy application, chemical adding or mixing. Some embodiments for the inflation material that may be used to provide outward pressure or a rigid structure from within the inflatable cuff 134 or network of inflatable channels 136 may include inflation materials formed from glycidyl ether and amine materials. Some inflation material embodiments may include an in situ formed hydrogel polymer having a first amount of diamine and a second amount of polyglycidyl ether wherein each of the amounts are present in a mammal or in a medical device, such as an inflatable graft, located in a mammal in an amount to produce an in situ formed hydrogel polymer that is biocompatible and has a cure time after mixing of about 10 seconds to about 30 minutes and wherein the volume of said hydrogel polymer swells less than 30 percent after curing and hydration. Some embodiments of the inflation material may include radiopaque material such as sodium iodide, potassium iodide, barium sulfate, Visipaque 320, Hypaque, Omnipaque 350, Hexabrix. For some inflation material embodiments, the polyglycidyl ether may be selected from trimethylolpropane triglycidyl ether, sorbitol polyglycidyl ether, polyglycerol polyglycidyl ether, pentaerythritol polyglycidyl ether, diglycerol polyglycidyl ether, glycerol polyglycidyl ether, trimethylolpropane polyglycidyl ether, polyethylene glycol diglycidyl ether, resorcinol diglycidyl ether, glycidyl ester ether of p-hydroxy benzoic acid, neopentyl glycol diglycidyl ether, 1,6-hexanediol diglycidyl ether, bisphenol A (PO)₂ diglycidyl ether, hydroquinone diglycidyl ether, bisphenol S diglycidyl ether, terephthalic acid diglycidyl ester, and mixtures thereof. For some inflation material embodiments, the diamine may be selected from (poly)alkylene glycol having amino or alkylamino termini selected from the group consisting of polyethylene glycol (400) diamine, di-(3-aminopropyl) diethylene glycol r, polyoxypropylenediamine, polyetherdiamine, polyoxyethylenediamine, triethyleneglycol diamine and mixtures thereof. For some embodiments, the diamine may be hydrophilic and the polyglycidyl ether may be hydrophilic prior to curing. For some embodiments, the diamine may be hydrophilic and the polyglycidyl ether is hydrophobic prior to curing. For some embodiments, the diamine may be hydrophobic and the polyglycidyl ether may be hydrophilic prior to curing.

The network of inflatable channels 136 may be partially or fully inflated by injection of a suitable inflation material into the main fill port 116 to provide rigidity to the network of inflatable channels 136 and the graft 114. In addition, a seal is produced between the inflatable cuff 134 and the inside surface of the abdominal aorta 10. Although it is desirable to partially or fully inflate the network of inflatable channels 136 of the graft 114 at this stage of the deployment process, such inflation step optionally may be accomplished at a later stage if necessary.

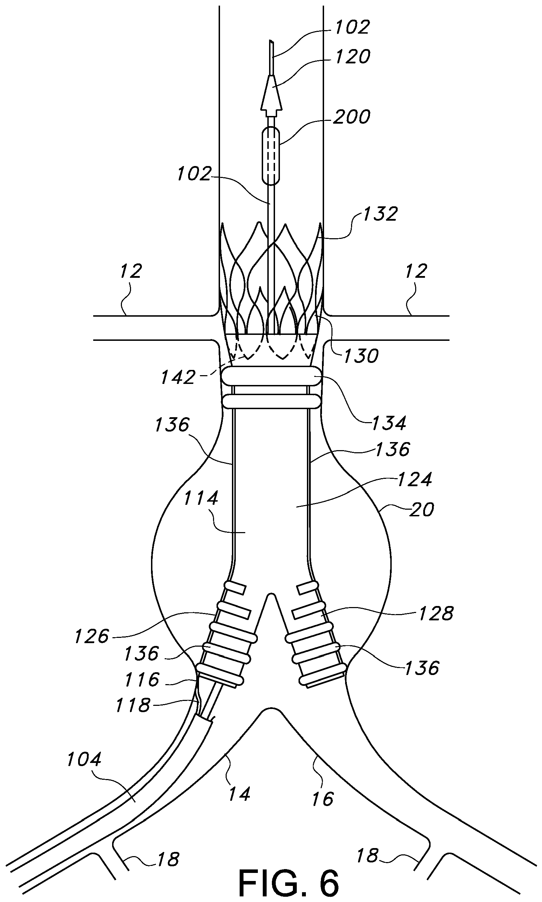

Once the graft 114 is deployed and the inflatable channels 136 thereof have been filled and expanded, the occlusion balloon 200 may be deflated, as depicted in FIG. 6. If desired, the occlusion balloon 200 may be repositioned (not shown) to occlude a portion of the prosthesis 106 or to further aid in the sealing of the inflatable cuff 134 against the inside surface of the abdominal aorta 10 or to further aid in the anchoring of the proximal portion 132 of the stent 108 with the inside surface of the patient's aorta 10. Moreover, the occlusion balloon 200 may be partially deflated or partially depressurized to allow, if desired, temporary perfusion of bodily fluid, such as blood, of bodily lumens or vessels otherwise being blocked by the previously inflated or pressurized occlusion balloon 200.

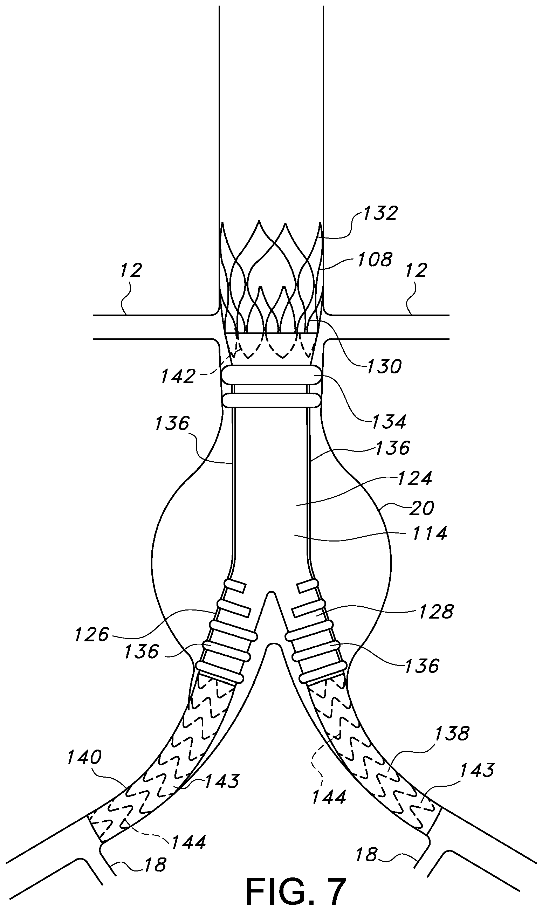

Once the graft 114 is deployed and the inflatable channels 136 thereof have been filled and expanded, another delivery catheter (not shown) may be used to deploy a contralateral graft extension 138, as depicted in FIG. 7. This is typically performed while the occlusion balloon 200 is deflated, but the present disclosure is not so limited, as the another delivery catheter could be advanced along the guidewire 102 with the occlusion balloon 200 being deflated, partially deflated, partially inflated and/or inflated. The contralateral graft extension 138 is in an axial position which overlaps the contralateral leg 128 of the graft 114. The amount of desired overlap of the graft extension 138 with the contralateral leg 128 may vary depending on a variety of factors including vessel morphology, degree of vascular disease, patient status. However, for some embodiments, the amount of axial overlap between the contralateral graft extension 138 and the contralateral leg 128 may be about 1 cm to about 5 cm; more specifically, about 2 cm to about 4 cm. Once the contralateral graft extension 138 has been deployed, an ipsilateral graft extension 140 may be deployed in the ipsilateral graft leg 126.

For some deployment embodiments, the patient's hypogastric arteries may be used to serve as a positioning reference point to ensure that the hypogastric arteries are not blocked by the deployment. Upon such a deployment, the distal end of a graft extension 138 or 140 may be deployed anywhere within a length of the ipsilateral leg 126 or contralateral leg 128 of the graft 114. Also, although only one graft extension 140, 138 is shown deployed on the ipsilateral side and contralateral side of the graft assembly 114, additional graft extensions 140, 138 may be deployed within the already deployed graft extensions 140, 138 in order to achieve a desired length extension of the ipsilateral leg 126 or contralateral leg 128. For some embodiments, about 1 to about 5 graft extensions 138, 140 may be deployed on either the ipsilateral or contralateral sides of the graft assembly 114. Successive graft extensions 138, 140 may be deployed within each other so as to longitudinally overlap fluid flow lumens of successive graft extensions. Such successive graft extensions are not limited to the use in treatment of AAA, but may also be used in other treatments, such as internal iliac preservation.

Graft extensions 138, 140, which may be interchangeable for some embodiments, or any other suitable extension devices or portions of the main graft section 124 may include a variety of suitable configurations. For some embodiments, graft extensions 138, 140 may include a polytetrafluoroethylene (PTFE) graft 143 with helical nitinol stent 144, although any suitable materials may be used for the graft and/or stent, if used.

Further details of the endovascular prosthesis 106 and/or graft extensions 138, 140 may be found in commonly owned U.S. Patent Nos. 6,395,019; 7,081,129; 7,147,660; 7,147,661; 7,150,758; 7,615,071; 7,766,954 and 8,167,927 and commonly owned U.S. Published Application No. 2009/0099649. Details for the manufacture of the endovascular prosthesis 106 may be found in commonly owned U.S. Patent Nos. 6,776,604; 7,090,693; 7,125,464; 7,147,455; 7,678,217 and 7,682,475. Useful inflation materials for the inflatable graft 114 may be found in may be found in commonly owned U.S. Published Application No. 2005/0158272 and 2006/0222596. Additional details concerning delivery details, including systems, devices and methods, of the ipsilateral graft leg 126 and the contralateral leg 128 may be found in commonly owned U.S. Published Application No. 2013/0338760. Additional details of an endovascular delivery system having an improved radiopaque marker system for accurate prosthesis delivery may be found in commonly owned U.S. Published Application No. 2013/0338752. Additional details of useful endovascular delivery systems and endovascular devices may be found in commonly owned U.S. Patent Nos. 6,602,280; 6,733,521; 6,761,733; 7,066,951; 7,241,276; 7,338,518; 7,901,379; 8,066,755; 8,083,789; 8,226,701; 8,328,861; and 8,663,309; the contents of all of which are incorporated in their entirety herein; in commonly owned U.S. Patent Application Publication Nos. 2003/0004560; 2004/0138734; 2006/0009833; 2009/0082841; 2009/0082845; 2009/0082846; 2009/0082847; 2009/0099649; 2009/0132026; 2010/0083870; 2011/0218609; 2012/0083870; 2012/0191174; 2013/0090715; 2013/0268044; 2013/0268048; 2014/0135899; and 2014/0350656 and in commonly owned International PCT Publication Nos. WO 2009/042789 A2; WO 2009/064923 A2; WO 2011/100367 A2; WO 2013/151896 A1; and WO 2013/151924 A1.

Useful graft materials for the endovascular prosthesis 106 include polyethylene; polypropylene; polyvinyl chloride; polytetrafluoroethylene (PTFE); fluorinated ethylene propylene; fluorinated ethylene propylene; polyvinyl acetate; polystyrene; poly(ethylene terephthalate); naphthalene dicarboxylate derivatives, such as polyethylene naphthalate, polybutylene naphthalate, polytrimethylene naphthalate and trimethylenediol naphthalate; polyurethane, polyurea; silicone rubbers; polyamides; polyimides; polycarbonates; polyaldehydes; polyether ether ketone; natural rubbers; polyester copolymers; silicone; styrene-butadiene copolymers; polyethers; such as fully or partially halogenated polyethers; and copolymers and combinations thereof. In some embodiments, the graft materials are non-textile graft materials, e.g., materials that are not woven, knitted, filament-spun, that may be used with textile grafts. Such useful graft material may be extruded materials. Particularly useful materials include porous polytetrafluoroethylene without discernable node and fibril microstructure and (wet) stretched PTFE layer having low or substantially no fluid permeability that includes a closed cell microstructure having high density regions whose grain boundaries are directly interconnected to grain boundaries of adjacent high density regions and having substantially no node and fibril microstructure , and porous PTFE having no or substantially no fluid permeability. Such PTFE layers may lack distinct, parallel fibrils that interconnect adjacent nodes of ePTFE, typically have no discernable node and fibril microstructure when viewed at a magnification of up to 20,000. A porous PTFE layer having no or substantially no fluid permeability may have a Gurley Number of greater than about 12 hours, or up to a Gurley Number that is essentially infinite, or too high to measure, indicating no measurable fluid permeability. Some PTFE layers having substantially no fluid permeability may have a Gurley Number at 100 cc of air of greater than about 10⁶ seconds. The Gurley Number is determined by measuring the time necessary for a given volume of air, typically, 25 cc, 100 cc or 300 cc, to flow through a standard 1 square inch of material or film under a standard pressure, such as 12.4 cm column of water. Such testing maybe carried out with a Gurley Densometer, made by Gurley Precision Instruments, Troy, NY. Details of such useful PTFE materials and methods for manufacture of the same may be found in commonly owned U.S. Patent Application Publication No. 2006/0233991.

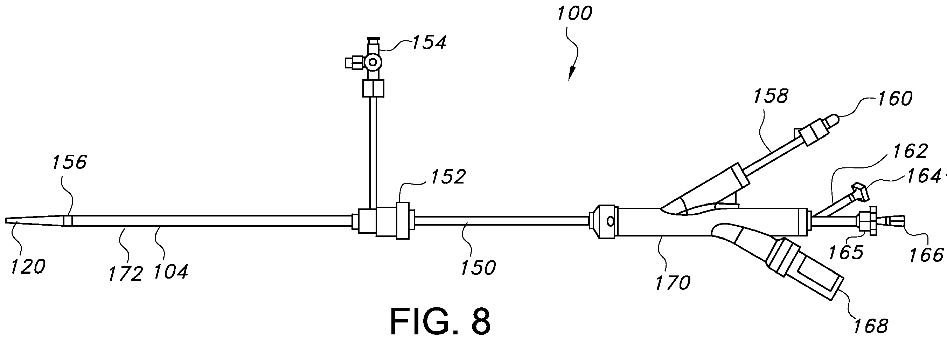

FIG. 8 is a side elevational view of an embodiment of the endovascular delivery system 100 of the present invention. The endovascular delivery system 100 may include, among other things, the nosecone 120; the outer sheath 104; a retraction knob or handle 152 for the outer sheath 104; a flush port 154 for the outer sheath 104; an outer sheath radiopaque marker band 156; an inner tubular member or hypotube 150; an inflation material or polymer fill connector port 158; an inflation material or polymer fill cap 160; a guidewire flush port 162; a guidewire flush port cap 164; a handle 165 for the inner tubular member 150; a guidewire port 166; and nested stent release knobs 168; interrelated as shown.

The flush port 154 for the outer sheath 104 may be used to flush the outer sheath 104 during delivery stages. The outer sheath 104 may have a radiopaque marker band to aid the practitioner in properly navigating the delivery system 100 to the desired bodily site. The outer sheath 104 is retractable by movement of the retraction knob or handle 152 for the outer sheath 104 by a practitioner towards the proximal handle assembly 170 of the delivery system 100. The inner tubular member or hypotube 150 is disposed from the inner tubular member or hypotube 150 toward a proximal portion of the delivery system 100. The inflation material or polymer fill connector port 158 and the inflation material or polymer fill cap 160 are useful for providing inflation material (e.g., polymeric fill material) to inflate proximal inflatable cuffs 134 and the network of inflatable channels 136 of the inflatable graft 114. The guidewire flush port 162 and the guidewire flush port cap 164 are useful for flushing the guidewire port 166 during delivery stages of the delivery system 100. The nested stent release knobs 168 contains a series of nested knobs (not shown) that that are used to engage release mechanisms for delivery of the endovascular prosthesis 106. Further details, including methods, catheters and systems, for deployment of endovascular prostheses are disclosed in commonly owned U.S. Patent Nos. 6,761,733 and 6,733,521 and commonly owned U.S. Patent Application Publication Nos. 2006/0009833 and 2009/0099649.

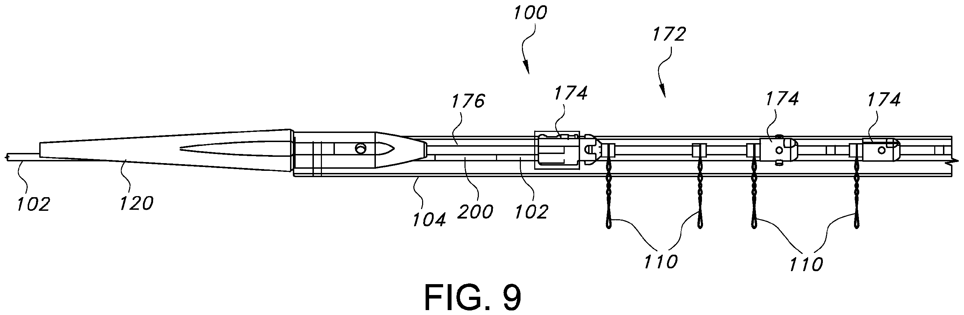

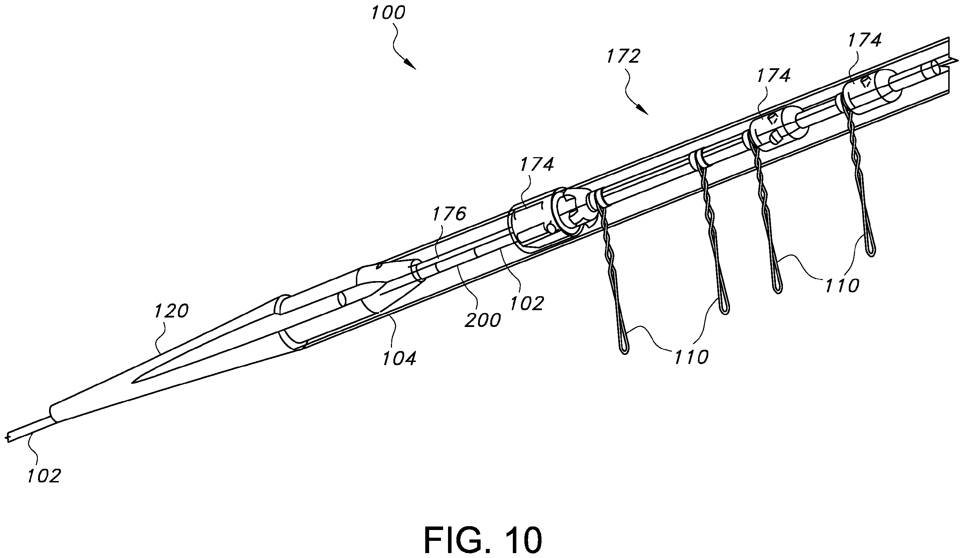

FIG. 9 is a side elevational and partial cutaway view of the distal portion 172 of an embodiment of an endovascular delivery system 100 of the present invention, and FIG. 10 is a partial perspective and partial cutaway view of the distal portion 172 of the endovascular delivery system 100 of the present invention. The distal portion 172 of the endovascular delivery system 100 includes prosthesis/stent holders 174 disposed upon a prosthesis/stent holder guidewire 176. The holders 174 are useful releasably securing the endovascular prosthesis 106 (not shown) within the delivery system 100. The holders 174 inhibit or substantially inhibit undesirable longitudinal and/or circumferential movement of the endovascular prostheses 106 during delivery stages of the delivery system 100. Belts 110 serve to restrain the endovascular prosthesis 106 in a radially constrained stage until desired release of the endovascular prosthesis 106.

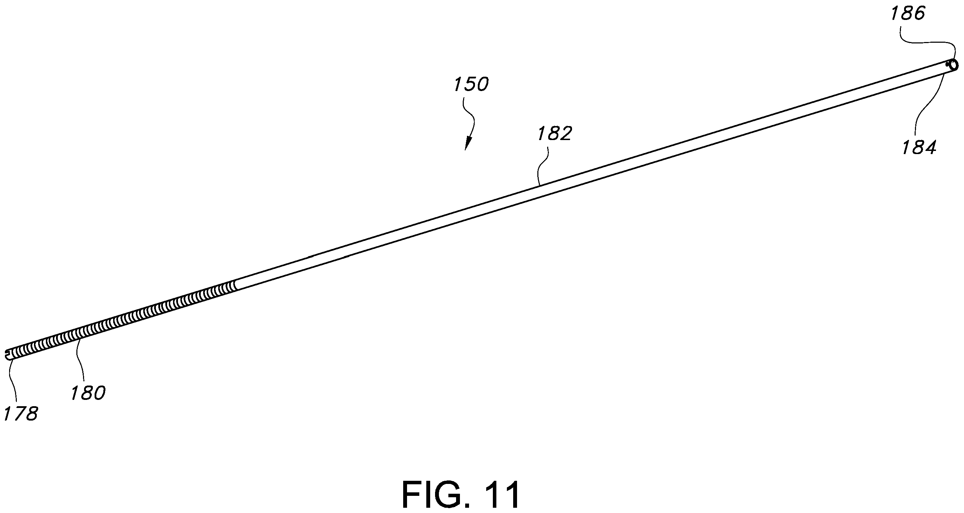

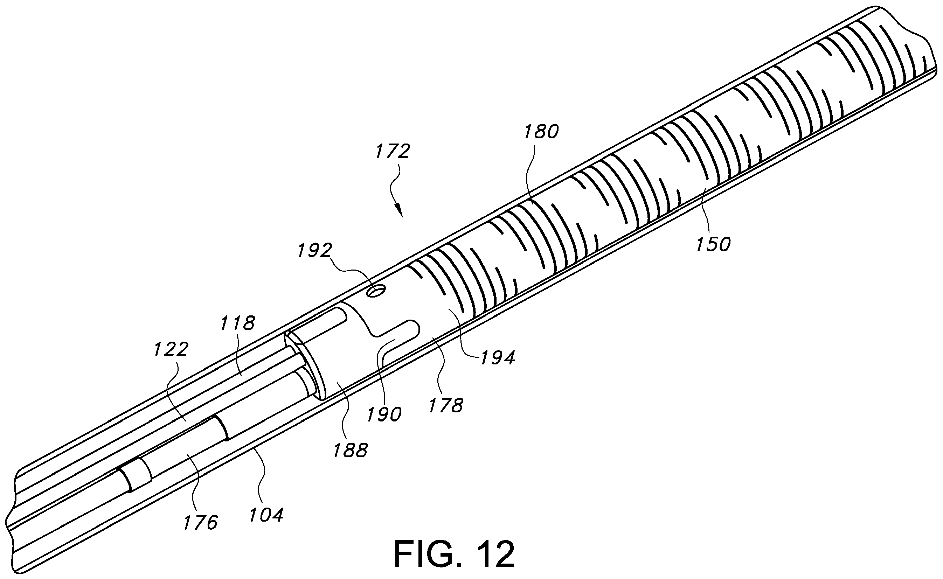

FIG. 11 is a perspective view of an embodiment of the inner tubular member 150, such as hypotube 150, of the endovascular delivery system 100 of the present invention, and FIG. 12 is a partial perspective and cutaway view of a distal portion 172 of an embodiment of the endovascular delivery system 100 of the present invention showing a distal end 178 of the hypotube 150. The hypotube has a hypotube wall 194, an open lumen 186 and opposed distal end 178 and proximal end 184 with a medial portion 182 therein between. The proximal end 184 of the hypotube 150 is securable disposed to the proximal handle assembly 170. The distal end 178 of the hypotube 150 has a cap 188 through which the inflation tube 118, the guidewire lumen 122 and the prosthesis/stent holders guidewire 176 passes there through i.e., from the proximal handle assembly 170 and through the open lumen 186 of the hypotube 150. The cap 188 may be made from any polymeric or plastic material. Polycarbonate is an example of one useful material for the cap 188. The guidewire lumen 122 may be made from polymeric materials such as polyimide, polyethylene, polyetheretherketone (PEEK), or other suitable polymers. The guidewire lumen 122 may have an outside diameter ranging from about 0.02 inches (0.51 mm) to about 0.08 inches (2.03 mm) and a wall thickness may range from about 0.002 inches (0.05 mm) to about 0.025 inches (0.64 mm). Other lumens disposed within the lumen 186 of the hypotube 150 may be made from other materials. The proximal and distal ends 184, 178 of the hypotube 150 have end slots 190 and end holes 192. Only end slots 190 and end holes 192 at the distal end 178 are depicted in FIG. 12. Such end slots 190 and end holes 192 are useful for securing the proximal end 184 of the hypotube 150 to the proximal handle assembly 170 and for securing the distal end 178 of the hypotube 150 to the distal cap 188 of the hypotube 150. An adhesive (not shown) may be inserted into end holes 192 to aid such securement. The end slots 190 are also useful for aligning the handle 170 to the device 100 such that the handle fill port 158 and the hypotube 150 are in the same plane but are offset at about 180° from each other.

One useful metallic material for the hypotube 150 may be 316 or 304 stainless steel. Other biocompatible materials may suitably be used, such as nitinol, cobalt-based alloy such as Elgiloy, platinum, gold, titanium, tantalum, niobium, and combinations thereof. The hypotube 150 may have a smooth exterior surface, such as one having a surface finish of less than or equal to about 32 microinches RMS. RMS is a measure of the smoothness of a surface. RMS refers to the Root Mean Square (RMS) of the average of measured peaks and valleys of a material surface profile calculated from a number of measurements along a sample length or area. Such RMS values are typically measured pursuant to ASTM D7127-015. RMS values from about 16 microinches to about 32 microinches are also useful.

Additional details concerning the hypotube 150 may be found in commonly owned U.S. Published Application No. 2013/0338753.

Typically, the occlusion balloon 200 will remain inflated until the endovascular prosthesis 106 is deployed. Further, if graft extension 138, 140 are to be deployed, then the occlusion balloon 200 will typically remain inflated during their deployment. The present disclosure, however, is not so limited. The occlusion balloon 200 may be inflated, may remain inflated, may be partially deflated, may be deflated or even may be re-inflated after deflation (partial or total) during the EVAR procedure as required or desired by a practitioner. For example, a practitioner may partially deflate or depressurize the occlusion balloon 200 after the main graft body 124 has been deployed so to allow temporary perfusion of the vessels, such as aorta 10, renal arteries 12, iliac arteries 14, 16, hypogastric arteries 18. Such partial depressurization allows temporary blood flow, but is not depressurized to an extent where hemostasis may be lost. After such partial depressurization of the occlusion balloon 200, the practitioner may move the occlusion balloon 200 distally a few centimeters, such as about 2, 3, 4, 5 cm or so, and re-inflate or repressurize the occlusion balloon 200 to continue occlusion while the remaining EVAR procedure is completed. The present disclosure, however, is not limited to partial depressurization of the occlusion balloon 200 followed by distal movement of the occlusion balloon 200. The occlusion balloon 200 may be kept in place after partial depressurization or, if desired, even moved proximally.

FIG. 13 depicts a portion of an embodiment of guidewire 102 of the present invention having an occlusion balloon 200. As depicted in FIG. 13, the occlusion balloon 200 is in an uninflated state or a delivery state. The embodiment of guidewire 102 shown is a hollow member having an open lumen portion 206, as depicted in FIG. 16. Inflation material (not shown) may be introduced through holes 204 of the guidewire 102. The present invention, however, is not limited to the use of a plurality holes 204 for providing ingress of inflation fluid to the balloon 200. Any useful porosity, for example fluid porosity, may be used for the ingress (and egress) of the inflation material.

Radiopaque markers 202 may be disposed on the guidewire 102 to aid in the visualization of the balloon under, for example, fluoroscopy. The radiopaque marker 202 (as well as other radiopaque markers described herein) may be a metallic band or a band of radiopaque material in, for example, a polymeric material. For example, a radiopaque marker may be made from a polymeric material which may also include radiopaque materials, such as metallic-based powders or ceramic-based powders, particulates or pastes which may be incorporated into the polymeric material. Various radiopaque materials and their salts and derivatives may be used including, without limitation, bismuth, barium and its salts such as barium sulfate, tantalum, tungsten, gold, platinum and titanium, to name a few. Metallic complexes useful as radiopaque materials are also contemplated.

While the optional radiopaque markers 202 are depicted as being on or associated with the guidewire 102 proximally before and after the occlusion balloon 202 in FIG. 13, the present invention is no so limited. For example, radiopaque markers may be disposed on or associated with the guidewire 102 over which the occlusion balloon 200 is disposed.

FIG. 14 is a partial exploded view of area or detail "A" of FIG. 13. The occlusion balloon 200 may include a balloon portion 201 at either or both ends of the occlusion balloon 200 that tapers towards the guidewire 102. Such a balloon portion 201 is useful for securing the occlusion balloon 200 to the guidewire 102. Such securing is typically in a fluid-tight manner at balloon inflation pressures. The balloon portion 201 may be secured to the guidewire 102 by any suitable technique, such as by adhesive or mechanical means. Mechanical means may include the use of, for example, a band of heat shrinkable material (not shown) or forming the balloon, for example by blow forming, directly onto the guidewire. As further described below, it is desirable that the occlusion balloon 200 and guidewire 102 assembly have a minimal profile, including a lower profile over commercially available occlusion balloons. Thus, the occlusion balloon 200 and guidewire 102 assembly of the present invention does not have a polymeric or plastic hollow base onto which the balloon is disposed and into which a guidewire is disposed and secured thereto.

Fig. 15 depicts an embodiment of the occlusion balloon 200 in an inflated or expanded state. The occlusion balloon 200 may be a compliant, a non-compliant, a semi-compliant and/or a compliant balloon. A compliant balloon is generally formed from elastomeric materials, such as latex, silicone polyurethane elastomers and nylon (e.g., polyamide) elastomers. A compliant balloon is typically formed by dip-molding elastomeric material into a tubular shape which can be expanded when inflated with an inflation material. Typical, but non-limiting, burst strength for a compliant balloon may be up to about 30 pounds per square inch (psi) (200 kilopascal or kPa). A non-compliant balloon is generally formed from thermoplastic materials, such as polyethylene terephthalate (PET), nylon and polyurethane thermoplastics. A non-compliant balloon is typically molded, dip-coated or blow formed to its "inflated" geometry to form the balloon. Typical, but non-limiting, burst strengths for a non-compliant balloon may be up to about 400 psi (2,900 kPa). A semi-compliant balloon is generally formed from thermoplastic, elastomeric materials, such as PET, nylon and polyurethane thermoplastics elastomers. A semi-compliant balloon is typically molded, dip-coated or blow formed to its "inflated" geometry to form the balloon. Typical, but non-limiting, burst strength for a semi-compliant balloon may be up to about 375 psi (2,600 kPa).

While the occlusion balloon 200 is depicted in FIG. 15 as being circular or spherical, including substantially circular or spherical, the present invention is not so limited. In general terms, the occlusion balloon 200, may include a body with two conical tapers and two necks at its proximal and distal ends. Either or both ends may include a conical sharp corner, conical radius corner, square end, spherical end, offset neck. Moreover, the balloon body may be a spherical balloon, a conical balloon, a square balloon, a conical/square balloon, a conical/square long balloon, a conical/spherical balloon, a long spherical balloon, a tapered balloon, a dog bone balloon, a stepped balloon. A rounder or more circular or spherical shape may be desired over these other non-spherical shapes, including those with longer axial lengths, so as to allow greater precision for the practitioner to control blood flow during pressurization, depressurization (partial or total), re-pressurization.

Commercial compliant balloons with elastomeric materials may include relatively thick walls of at least about 0.015 inches (0.38 mm). Commercial non-compliant and semi-compliant balloons often include wall thicknesses of at least about several thousands of an inch, such as 0.005 inches (0.13 mm).

The occlusion balloon 200 of the present invention may be a compliant, semi-compliant or non-compliant with a wall thickness of about 0.001 inches (0.025 mm). Slightly larger or slightly smaller wall thicknesses may be used, such as about 0.0005 inches (0.01 mm) to about 0.003 inches (0.08 mm). The occlusion balloon 200 of the present invention typically is sized or initially formed at a size larger than the target bodily lumen, such as aorta 10.

Several standard commercial aortic occlusion balloons are Cook's Coda® and Medtronic's Reliant® balloons. The Cook recommends a 12 French (4 mm) introducer for the 32 mm balloon and a 14 French (4.7 mm) introducer for the 40 mm balloon for its Coda® balloons. Medtronic recommends a 12 French (4 mm) introducer for the up to 46 mm balloon for its Reliant® balloons. These balloons are too large in delivery diameter to fit into the delivery system 100 of the present invention. Indeed, the delivery catheter shaft itself is 9 French (3 mm) for the Coda® balloon and 8 French (2.7 mm) for the Reliant® balloon.

In order to reduce the balloon catheter device to a 10 French introducer, the delivery shaft under the balloon may also be reduced which reduces the cross-section of the inflation/deflation lumen resulting in longer inflation and deflation times. For example, a smaller (7 French - 7.5 French) balloon mounting shaft may be used to obtain such a 10 French introducer compatibility. This reduces the pressure drop along the length of the delivery catheter in order to maintain the rapid inflation/deflation times of the balloon in order to maintain competitiveness with existing balloons.

The guidewire 102 of the present invention has an outer diameter of about 0.035 inches (0.9 mm) or about 3 French. This diameter is not limiting and may vary from about 0.025 inches (0.64 mm) to about 0.045 inches (1.1 mm) depending upon the French size of the delivery system 100 of the present invention. The length of the guidewire 102 with the occlusion balloon 200 is typically long enough to accommodate the delivery system, such as endovascular delivery system 100. A total, non-limiting, length may be about 150 cm to about 180 cm, but other lengths may suitable be used. Moreover, the guidewire 102 may have varying stiffness along its length. For example, the guidewire 102 may be stiffer at a location distal to the occlusion balloon 200. This may be desirable for situations where more column strength is needed to support the occlusion balloon 200 when the occlusion balloon 200 needs to be moved, for example moved proximally for anatomical reasons, procedural reasons. Such increased stiffness may be accomplished by any suitable means, such as the use of more material, such as a larger diameter guidewire portion, the use of material that is inherently stiffer than other portions of the guidewire 102, the use of composite material with greater stiffness. A non-limiting increased wire diameter portion may include a portion with a diameter of about 0.04 inches (1.02 mm).

As depicted in the embodiment of Fig 16, which is a cross-sectional and perspective view taken along the B-B axis of FIG. 15, guidewire 102 of the present invention has a hollow lumen 206 extending through at least a portion of the guidewire 102. The open lumen 206 may have an open lumen diameter from about 0.015 inches (0.38 mm) to about 0.030 inches (0.76 mm) for a guidewire 102 having an outer diameter of about 0.035 inches (0.9 mm). The distal portion of the guidewire 102 may be open or closed. The guidewire may be made from any suitable metal, such as stainless steel or nitinol. The present invention, however, is not limited to the use of metallic materials for the guidewire 102. For example, the guidewire 102 may be a polymeric, either in total or in part, guidewire. Useful, but non-limiting polymeric materials include polyetheretherketone (PEEK), polyamide.

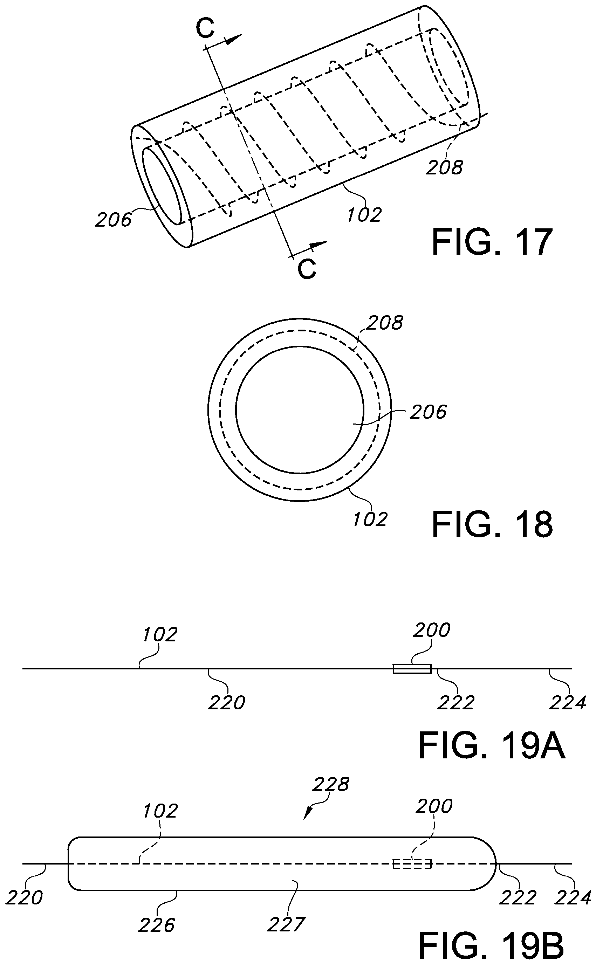

As depicted in FIGS. 17 and 18, an embodiment of a polymeric guidewire 102 may include a reinforcement or support coil 208. Such a reinforcing coil 208 may be in for form of a metallic coil, such as stainless steel or nitinol. The present invention is not limited to the use of a reinforcing coil 208 with a polymeric guidewire 102. The polymeric guidewire 102 may include other reinforcing means, such as reinforcing fibers or particulates, including carbon fibers or particulates. Further, the polymeric guidewire 102 may be free or not include the reinforcing coil 208.

FIG. 19A is a side elevational view of an embodiment of the occlusion balloon 200 and guidewire 102 assembly of the present invention. The guidewire 102 includes a proximal portion 220, a distal portion 222 and a distal end portion 224. The distal portion 222 and the distal end portion 224 may be the same or different.

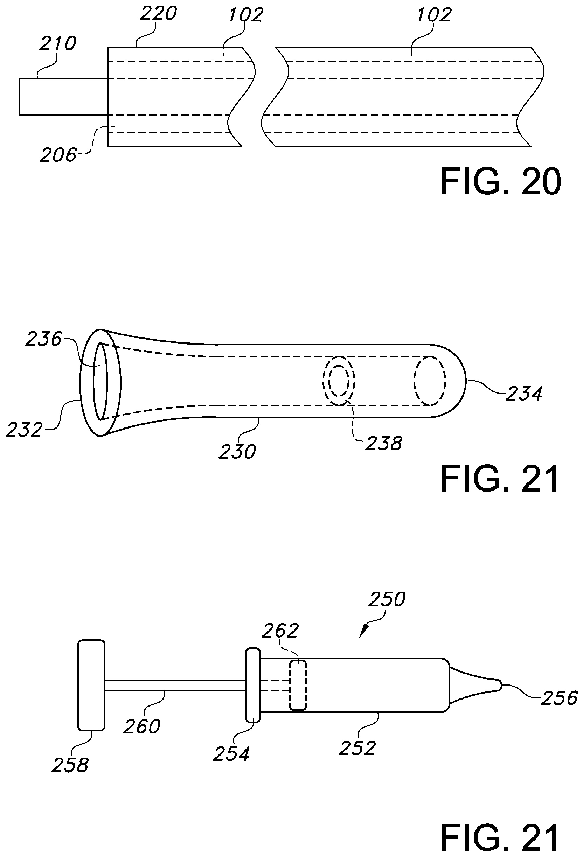

As depicted in an embodiment of FIG. 20, a removable mandrel 210 may be slidably disposed within the lumen 206 of the guidewire 102. Such a removable mandrel 210 may be used for both a metallic and/or polymeric hollow guidewire 102. The removable mandrel 210 is useful in providing stiffness to the occlusion balloon 200 and the guidewire 102 assembly during deployment is useful in avoiding kinking of the guidewire 102 during deployment. Although there is a space depicted in FIG. 20 between the removable mandrel 210 and the lumen 206 of the guidewire 102, such a space is an example. Such a space should be minimized to maximize stiffness and avoid kinking. Frictional forces between the removable mandrel 210 and the wall of the lumen 206 of the guidewire 102 should be minimized. A low friction material may be applied to the removable mandrel 210 and/or the removable mandrel 210 may be imparted with a smooth surface, such as a surface finish of less than or equal to about 32 micro inches RMS.

FIG. 21 depicts an embodiment of a luer fitting 230 or luer-type fitting useful with the present invention. Such a luer fitting 230 may be removably disposed at a handle, such as handle 165, of the proximal handle assembly 170. The luer fitting 230 has a proximal open end 232 and an opposed open distal end 234 with an open lumen 236. The guidewire 102 (not shown) may be slidably disposed within the open lumen 236 of the luer fitting 230. The luer fitting 230 may include a sealing ring or O-ring 238 to prevent fluid flow, such as balloon inflation material (not shown), over the guidewire 102 and out of the proximal open end 232 of the luer fitting 230.

The luer fitting 230 is useful for inflating or deflating the occlusion balloon 200. The occlusion balloon 200 may be inflated and deflated with a syringe or other suitable device. A useful syringe 250 is depicted in FIG. 250. The syringe 250 may include a syringe barrel 252 having or even free of a syringe flange 254, an open tip 256, a plunger 258, a plunger stem 260 and a piston 262, inter-related as shown. As compared to conventional or commercially available balloons, the inflation lumen of hollow lumen 206 of the guidewire 102 is considerably smaller for the occlusion balloon 200, as described above. To achieve desirably balloon inflation times, relatively small syringes are used as small syringes may have a higher fluid delivery pressure as compared to larger syringes. Thus, higher hand injection pressures may be achieved with the small syringes of the present invention. Procedurally, two small syringes (e.g. 20 cc) may be used. The first one may be used to partially fill the occlusion balloon 200, and the second one may be used to reach the desired occlusion volume. The use of two such syringes has achieved 15 to 30 second inflation times through small open lumens, such as 0.030 inches (0.076 mm). Such quick inflation times are important to avoid potential blood loss due from, for example, a rupture of an aneurysm. Balloon deflation time is not as critical and can take longer. A larger syringe, such as 40 cc may be used to evacuate the occlusion balloon 200. Another important aspect in achieving quick inflation times with small inflation lumens or guidewire lumen 206 of the present invention is through the use of a balloon inflation material specifically tailored for such small lumens and quick inflation times. Inflation materials for commercially available balloons use considerable contrast materials, typically 50% to 75% or more, in their inflation materials. The present invention may use a more dilute contrast/saline mixture. For example, inflation materials useful with the present invention may suitably include only 5% to 10% contrast material in saline. Useful, but not limiting contrast material include radiopaque materials such as sodium iodide, potassium iodide, barium sulfate, Visipaque 320, Hypaque, Omnipaque 350, Hexabrix.

While the occlusion balloon 200 and the guidewire 102 assembly, such as depicted in FIG. 19A, have been described for use with the delivery system 100 for deploying the prosthesis 106, the present invention is not so limited. For example, a catheter 226 having a sheath 227 may be used to deploy the occlusion balloon 200 and the guidewire 102 assembly (228) without the deployment system 100 being used. Such a catheter 226 may be useful in inflating the occlusion balloon 200 in rapid endovascular balloon occlusion (REBO) techniques. Such REBO techniques may be used in unstable patients with ruptured aneurysms or even on in non-aneurysmal situations, for example in trauma situations, including civilian and military, to stop undue bleeding and to stabilize a patient.

In either a REBO situation or in a prosthesis delivery situation, the guidewire 102 may include a distal end portion 224 having reduced stiffness and greater flexibility, such as a floppy tip portion. The overall length of the distal portion 222 (i.e., from the distal portion of the balloon to the distal end of the guidewire) of the guidewire may be from about 20 cm to about 30 cm. The first about 5 cm to about 10 cm of the distal portion 222 proximal to the balloon 200 may be stiff for delivery considerations, thereafter followed by a floppy tip or end portion. A floppy tip may especially be useful in REBO applications, where the need for rapid deployment of the balloon 200 may be critical and where the floppy tip will minimize any additional trauma in such rapid deployment. The proximal portion 220 of the guidewire 102 may vary in length. For example the proximal portion 220 may vary from about 60 cm to 100 cm or more. The overall length of the guidewire 102 may be from 100 cm to 120 cm. The longer the guidewire length, the greater pressure drop or resistance to fluid flow, i.e., inflation material, will be present. To provide flexibility, for example in REBO situations, then proximal portion 220 of the guidewire 102 may be segmented such that its length man be easily changed in critical trauma situations to provide for as rapid balloon inflation times as possible. The above dimensional lengths for the guidewire and guidewire portions are non-limiting, and other lengths may suitably be used. In an embodiment of a REBO application, the balloon 200 may be a non-compliant or semi-compliant balloon. As described above, in an embodiment of an EVAR application, the balloon 200 may be a non-compliant, semi-compliant or compliant balloon.

The following embodiments or aspects of the disclosure may be combined in any fashion and combination and be within the scope of the present disclosure, as follows:
Embodiment 1. An endovascular delivery system (100), comprising:
   a prosthesis (106) comprising a main tubular body (124) having an open proximal end and opposed open ipsilateral and contralateral legs (126, 128);
   an elongate outer tubular sheath (104) having an open lumen and opposed proximal and distal ends with a medial portion therein between;
   an elongate inner tubular member (150) slidably disposed within the open lumen of the outer tubular sheath (104); wherein the distal end of the outer tubular sheath (104) being slidably disposed past and beyond the distal end of the inner tubular member (150) to define a prosthesis delivery state and slidably retractable to the medial portion of the inner tubular member (150) to define a prosthesis unsheathed state;
   an elongate guidewire (102) slidably disposed within the inner tubular member (150) and extending from the handle assembly (170), through the ipsilateral leg (126) of the prosthesis (106) and through the main tubular body (124) of the prosthesis (106) and extending past the open of the main tubular body (124) in the prosthesis delivery state; and
   an inflatable occlusion balloon (200) disposed on a portion of the elongate guidewire (102) extending past the open end of the main tubular body (124);
   wherein the elongate guidewire (102) comprises a hollow portion (206) such that the inflatable occlusion balloon (200) is in fluid communication with a balloon inflation material.
Embodiment 2. The endovascular delivery system (100) of Embodiment 1, wherein said main tubular body (124) comprising an inflatable cuff (134) disposed near the open proximal end.
Embodiment 3. The endovascular delivery system (100) of Embodiment 2, wherein the portion of the elongate guidewire (102) over which the occlusion balloon (200) is disposed has porosity (204) for ingress and egress of the balloon inflation material.
Embodiment 4. The endovascular delivery system (100) of Embodiment 3, wherein the porosity (204) is a plurality of holes (204) disposed through the elongate guidewire (102).
Embodiment 5. The endovascular delivery system (100) of Embodiment 2, wherein the balloon inflation material comprises saline and contrast material.
Embodiment 6. The endovascular delivery system (100) of Embodiment 1, wherein the elongate guidewire (102) comprises a metallic material.
Embodiment 7. The endovascular delivery system (100) of Embodiment 1, wherein the elongate guidewire (102) comprises a polymeric material.
Embodiment 8. The endovascular delivery system (100) of Embodiment 7, further comprising a removable mandrel (210) disposed within the elongate guidewire (102) for supporting elongate guidewire (102) prior to inflation of the occlusion balloon (200).
Embodiment 9. The endovascular delivery system (100) of Embodiment 1, further comprising a seal (212) disposed on a proximal portion of the elongate guidewire (102), such as a portion disposed within the handle assembly (170).
Embodiment 10. The endovascular delivery system (100) of Embodiment 1, wherein said ipsilateral and contralateral legs (126, 128) comprise inflatable channels (136).
Embodiment 11. The endovascular delivery system (100) of Embodiment 1, further comprising a first radiopaque marker (202) disposed on a portion of the elongate guidewire (102) just past the occlusion balloon (200).
Embodiment 12. The endovascular delivery system (100) of Embodiment 11, further comprising a second radiopaque marker (202) disposed on a portion of the elongate guidewire (102) just prior the occlusion balloon (200).
Embodiment 13. An endovascular delivery system (100), comprising:
   a prosthesis (106) comprising a main tubular body (124) having an open end and opposed open end;
   an elongate outer tubular sheath (104) having an open lumen and opposed proximal and distal ends with a medial portion therein between;
   an elongate inner tubular member (150) slidably disposed within the open lumen of the outer tubular sheath (104); wherein the distal end of the outer tubular sheath (104) being slidably disposed past and beyond the distal end of the inner tubular member (150) to define a prosthesis delivery state and slidably retractable to the medial portion of the inner tubular member (150) to define a prosthesis unsheathed state;
   an elongate guidewire (102) slidably disposed within the inner tubular member (150) and extending from the handle assembly (170) through the main tubular body (124) of the prosthesis (106) and extending past the open of the main tubular body (124) in the prosthesis delivery state; and
   an inflatable occlusion balloon (200) disposed on a portion of the elongate guidewire (102) extending past the open end of the main tubular body (124).
Embodiment 14. The endovascular delivery system (100) of Embodiment 13, wherein the prosthesis (106) is a bifurcated prosthesis having open ipsilateral and contralateral legs (126, 128) at the opposed open end.
Embodiment 15. A method for delivering a bifurcated prosthesis, comprising:
   providing the endovascular delivery system (100) of Embodiment 1;
   advancing the endovascular delivery system (100) through a first branched artery (14) and into an aneurysm (20) in a main artery (10);
   retracting the outer sheath (104) so that the proximal end of the main tubular body (124) of the prosthesis (106) is disposed beyond the aneurysm (20) and so that the ipsilateral and contralateral legs (126, 128) are disposed within the aneurysm (20);
   inflating the occlusion balloon (200) with an inflation material in the main artery (10) beyond the aneurysm (20) to provide a seal against blood flow thereat;
   deploying the prosthesis (106); and
   deflating the occlusion balloon (200).
Embodiment 16. The method of Embodiment 15 further comprising:
   deploying a contralateral graft extension (138) having opposed proximal and distal open ends contained within a catheter so that the proximal end of the contralateral graft extension (138) is disposed within a portion of the contralateral leg (128) of the main tubular body (124) of the prosthesis (106) and so that the distal end of the contralateral graft extension (138) is disposed distally of the aneurysm (20) and within a portion of a second branched artery (16).
Embodiment 17. The method of Embodiment 15 further comprising:
   deploying a ipsilateral graft extension (140) having opposed proximal and distal open ends contained within a second catheter so that the proximal end of the ipsilateral graft extension (140) is disposed within a portion of the ipsilateral leg (126) of the main tubular body (124) of the prosthesis (106) and so that the distal end of the ipsilateral graft extension (140) is disposed distally of the aneurysm (20) and within a portion of the first branched artery (14).
Embodiment 18. An assembly (228) for rapid endovascular balloon occlusion, comprising:
   a guidewire (102) having a hollow lumen portion (206), a proximal portion (220), a distal portion (222) and a balloon mounting portion therein between;
   a non-compliant or semi-compliant occlusion balloon (200) securably disposed on the balloon mounting portion of the guidewire (102) and in fluid communication with the hollow lumen portion (206); and
   a catheter (226) having a sheath (227) having the non-compliant or semi-compliant balloon (200) and portions of the guidewire (102) slidably disposed therein.
Embodiment 19. The assembly (228) of Embodiment 18, further comprising:
   a syringe (250) having inflation material for inflating the non-compliant or semi-compliant balloon (200); and a luer fitting (230) for providing fluid communication of the inflation material to the hollow lumen portion (206) of the guidewire (102).
Embodiment 20. The assembly (228) of Embodiment 18, wherein the guidewire (102) has an outer diameter of about 0.035 inches and a hollow lumen diameter of about 0.015 inches to about 0.030 inches.
Embodiment 21. A method for rapid endovascular balloon occlusion, comprising:
   providing a guidewire (102) having a hollow lumen portion (102), a proximal portion (220), a distal portion (224) and a balloon mounting portion therein between;
   providing a non-compliant or semi-compliant occlusion balloon (200) securably disposed on the balloon mounting portion of the guidewire (102) and in fluid communication with the hollow lumen portion (206);
   providing a catheter (226) having a sheath (227) having the non-compliant or semi-compliant balloon (200) and portions of the guidewire (102) slidably disposed therein;
   providing a syringe (250) having inflation material for inflating the non-compliant or semi-compliant balloon (200); and a luer fitting (230) for providing fluid communication of the inflation material to the hollow lumen portion (206) of the guidewire (102);
   delivering the catheter (226) to a desired bodily location;
   withdrawing the sheath (227) of the catheter (226) to expose the occlusion balloon (200); and
   inflating the occlusion balloon (200) with the inflation material.
Embodiment 22. The method of Embodiment 21, wherein the occlusion balloon (200) is inflated with the inflation material within about 15 to about 30 seconds.
Embodiment 23. The method of Embodiment 21, wherein the guidewire (102) has an outer diameter of about 0.035 inches and a hollow lumen diameter of about 0.015 inches to about 0.030 inches.

While various embodiments of the present invention are specifically illustrated and/or described herein, it will be appreciated that modifications and variations of the present invention may be effected by those skilled in the art. Further, any of the embodiments or aspects of the invention as described in the claims or in the specification may be used with one and another without limitation.

## Claims

1. An endovascular delivery system (100), comprising:
a prosthesis (106) comprising a main tubular body (124) having an open proximal end and opposed open ipsilateral and contralateral legs (126, 128);
an elongate outer tubular sheath (104) having an open lumen and opposed proximal and distal ends with a medial portion therein between;
an elongate inner tubular member (150) slidably disposed within the open lumen of the outer tubular sheath (104); wherein the distal end of the outer tubular sheath (104) being slidably disposed past and beyond the distal end of the inner tubular member (150) to define a prosthesis delivery state and slidably retractable to the medial portion of the inner tubular member (150) to define a prosthesis unsheathed state;
an elongate guidewire (102) slidably disposed within the inner tubular member (150) and extending from the handle assembly (170), through the ipsilateral leg (126) of the prosthesis (106) and through the main tubular body (124) of the prosthesis (106) and extending past the open of the main tubular body (124) in the prosthesis delivery state; and
**characterized in that** the endovascular delivery system (100) comprises
an inflatable occlusion balloon (200) disposed on a portion of the elongate guidewire (102) extending past the open end of the main tubular body (124);
wherein the elongate guidewire (102) comprises a hollow portion (206) such that the inflatable occlusion balloon (200) is in fluid communication with a balloon inflation material;
wherein the endovascular delivery system (100) has an overall outer diameter from about 3.3 mm (10 French) to about 4.7 mm (14 French) or less than 3.3 mm (10 French);
wherein the inflatable occlusion balloon (200) has a wall having a thickness from about 0.01 mm (0.0005 inches) to about 0.08 mm (0.003 inches); and
wherein the elongate guidewire (102) an outer diameter from about 0.64 mm (0.025 inches) to about 1.1 mm (0.045 inches).

2. The endovascular delivery system (100) of claim 1, wherein said main tubular body (124) comprising an inflatable cuff (134) disposed near the open proximal end.

3. The endovascular delivery system (100) of any one of the preceding claims, wherein the portion of the elongate guidewire (102) over which the occlusion balloon (200) is disposed has porosity (204) for ingress and egress of the balloon inflation material.

4. The endovascular delivery system (100) of claim 3, wherein the porosity (204) is a plurality of holes (204) disposed through the elongate guidewire (102).

5. The endovascular delivery system (100) of any one of the preceding claims, wherein the balloon inflation material comprises saline and contrast material.

6. The endovascular delivery system (100) of any one of the preceding claims, wherein the elongate guidewire (102) comprises a metallic material.

7. The endovascular delivery system (100) of any one of claims 1-5, wherein the elongate guidewire (102) comprises a polymeric material.

8. The endovascular delivery system (100) of any one of the preceding claims, further comprising a removable mandrel (210) disposed within the elongate guidewire (102) for supporting elongate guidewire (102) prior to inflation of the occlusion balloon (200).

9. The endovascular delivery system (100) of any one of the preceding claims, further comprising a seal (212) disposed on a proximal portion of the elongate guidewire (102), such as a portion disposed within the handle assembly (170).

10. The endovascular delivery system (100) of any one of the preceding claims, wherein said ipsilateral and contralateral legs (126, 128) comprise inflatable channels (136).

11. The endovascular delivery system (100) of any one of the preceding claims, further comprising a first radiopaque marker (202) disposed on a portion of the elongate guidewire (102) just past the occlusion balloon (200).

12. The endovascular delivery system (100) of claim 11, further comprising a second radiopaque marker (202) disposed on a portion of the elongate guidewire (102) just prior the occlusion balloon (200).

13. The endovascular delivery system (100) of any one of the preceding claims, further comprising:
a syringe (250) having inflation material for inflating the inflatable occlusion balloon (200); and a luer fitting (230) for providing fluid communication of the inflation material to the hollow lumen portion (206) of the elongate guidewire (102).

14. The endovascular delivery system (100) of any one of the preceding claims, wherein the guidewire (102) has an outer diameter of about 0.89 mm (0.035 inches) and a hollow lumen diameter from about 0.38 mm (0.015 inches) to about 0.76 mm (0.030 inches).

15. The endovascular delivery system (100) of any one of claims 1-6 and 8-12, wherein the elongate guidewire (102) comprises a polymeric material to define a polymeric guidewire (102), wherein the polymeric guidewire (102) comprises a reinforcement coil (208) or reinforcing fibers or particulates.

## Patentansprüche

1. Endovaskuläres Abgabesystem (100), umfassend:
ein Prothese (106), umfassend einen röhrenförmigen Hauptkörper (124), der ein offenes proximales Ende und entgegengesetzte offene ipsilaterale und kontralaterale Beine (126, 128) aufweist;
eine längliche äußere röhrenförmige Hülle (104), die ein offenes Lumen und gegenüberliegende proximale und distale Enden mit einem Mittelteil dazwischen aufweist;
ein längliches inneres röhrenförmiges Element (150), das verschiebbar in dem offenen Lumen der äußeren röhrenförmigen Hülle (104) disponiert ist; wobei das distale Ende der äußeren röhrenförmigen Hülle (104) verschiebbar über dem und über das distale Ende des inneren röhrenförmigen Elements (150) hinaus disponiert ist, um einen Prothesen-Verabreichungszustand zu definieren, und verschiebbar in den Mittelteil des inneren röhrenförmigen Elements (150) einziehbar ist, um einen aus der Hülle gezogenen Prothesenzustand zu definieren;
einen länglichen Führungsdraht (102), der verschiebbar in dem inneren röhrenförmigen Element (150) disponiert ist und sich aus der Griffeinheit (170) durch das ipsilaterale Bein (126) der Prothese (106) und durch den röhrenförmigen Hauptkörper (124) der Prothese (106) erstreckt und sich über die Öffnung des röhrenförmigen Hauptkörpers (124) im Prothesen-Verabreichungszustand hinaus erstreckt; und
**dadurch gekennzeichnet, dass** das endovaskuläre Abgabesystem (100) einen aufblasbaren Okklusionsballon (200) aufweist, der an einem Abschnitt des länglichen Führungsdrahtes (102) disponiert ist, der über das offene Ende des röhrenförmigen Hauptkörpers (124) hinausragt;
wobei der längliche Führungsdraht (102) einen hohlen Abschnitt (206) aufweist, so dass der aufblasbare Okklusionsballon (200) in Fluidverbindung mit einem Ballonaufblasmaterial steht;
wobei das endovaskuläre Abgabesystem (100) einen Gesamtaußendurchmesser von etwa 3,3 mm (10 French) bis ca. 4,7 mm (14 French) oder weniger als 3,3 mm (10 French) hat;
wobei der aufblasbare Okklusionsballon (200) eine Wand mit einer Dicke von etwa 0,01 mm (0,0005 Zoll) bis etwa 0,08 mm (0,003 Zoll) aufweist; und
wobei der längliche Führungsdraht (102) einen Außendurchmesser von etwa 0,64 mm (0,025 Zoll) bis etwa 1,1 mm (0,045 Zoll) hat.

2. Endovaskuläres Abgabesystem (100) nach Anspruch 1, wobei der röhrenförmige Hauptkörper (124) eine aufblasbare Manschette (134) umfasst, die in der Nähe des offenen proximalen Endes disponiert ist.

3. Endovaskuläres Abgabesystem (100) nach einem der vorhergehenden Ansprüche, wobei der Abschnitt des länglichen Führungsdrahtes (102), über dem der Okklusionsballon (200) disponiert ist, Porosität (204) für das Eintreten und Austreten von Ballonaufblasmaterial aufweist.

4. Endovaskuläres Abgabesystem (100) nach Anspruch 3, wobei die Porosität (204) eine Vielzahl von Löchern (204) aufweist, die durch den länglichen Führungsdraht (102) disponiert sind.

5. Endovaskuläres Abgabesystem (100) nach einem der vorhergehenden Ansprüche, wobei das Ballonaufblasmaterial Kochsalzlösung und Kontrastmittel umfasst.

6. Endovaskuläres Abgabesystem (100) nach einem der vorhergehenden Ansprüche, wobei der längliche Führungsdraht (102) metallisches Material umfasst.

7. Endovaskuläres Abgabesystem (100) nach einem der Ansprüche 1 - 5, wobei der längliche Führungsdraht (102) ein Polymer-Material umfasst.

8. Endovaskuläres Abgabesystem (100) nach einem der vorhergehenden Ansprüche, das ferner einen entfernbaren Dorn (210) aufweist, der im länglichen Führungsdraht (102) zur Unterstützung des länglichen Führungsdrahtes (102) vor dem Aufblasen des Okklusionsballons (200) angeordnet ist.

9. Endovaskuläres Abgabesystem (100) nach einem der vorhergehenden Ansprüche, das ferner eine Dichtung (212) umfasst, die an einem proximalen Abschnitt des länglichen Führungsdrahtes (102) derart angeordnet ist, dass ein Abschnitt innerhalb der Griffeinheit (170) disponiert ist.

10. Endovaskuläres Abgabesystem (100) nach einem der vorhergehenden Ansprüche, wobei die ipsilateralen und kontralateralen Beine (126, 128) aufblasbare Kanäle (136) umfassen.

11. Endovaskuläres Abgabesystem (100) nach einem der vorhergehenden Ansprüche, das ferner eine erste röntgendichte Markierung (202) umfasst, die an einem Abschnitt des länglichen Führungsdrahtes (102) direkt hinter dem Okklusionsballon (200) disponiert ist.

12. Endovaskuläres Abgabesystem (100) nach Anspruch 11, das ferner eine zweite röntgendichte Markierung (202) umfasst, die an einem Abschnitt des länglichen Führungsdrahtes (102) direkt vor dem Okklusionsballon (200) disponiert ist.

13. Endovaskuläres Abgabesystem (100) nach einem der vorhergehenden Ansprüche, ferner umfassend:
eine Injektionsspritze (250) mit Aufblasmaterial zum Aufblasen des aufblasbaren Okklusionsballons (200) und einem Luer-Anschluss (230) für die Bereitstellung der Fluidkommunikation des Aufblasmaterials mit dem hohlen Lumenabschnitt (206) des länglichen Führungsdrahtes (102).

14. Endovaskuläres Abgabesystem (100) nach einem der vorhergehenden Ansprüche, wobei der Führungsdraht (102) einen Außendurchmesser von etwa 0,89 mm (0,035 Zoll) und einen Hohllumendurchmesser von etwa 0,38 mm (0,015 Zoll) bis ca. 0,76 mm (0,030 Zoll) aufweist.

15. Endovaskuläres Abgabesystem (100) nach einem der Ansprüche 1 - 6 und 8 - 12, wobei der längliche Führungsdraht (102) ein Polymer-Material umfasst, um einen Polymer-Führungsdraht (102) zu definieren, wobei der Polymer-Führungsdraht (102) eine Verstärkungsspirale (208) oder Verstärkungsfasern oder -partikel umfasst.

## Revendications

1. Système de pose endovasculaire (100) comprenant :
une prothèse (106) comprenant un corps tubulaire principal (124) ayant une extrémité proximale ouverte et des pattes de ipsilatérale et contralatérale (126, 128) ouvertes opposées ;
une gaine tubulaire externe allongée (104) ayant une lumière ouverte et des extrémités proximale et distale opposées avec une partie médiale entre elles ;
un élément tubulaire interne allongé (150) disposé, de manière coulissante, à l'intérieur de la lumière ouverte de la gaine tubulaire externe (104) ; dans lequel l'extrémité distale de la gaine tubulaire externe (104) est disposée, de manière coulissante, après et au-delà de l'extrémité distale de l'élément tubulaire interne (150) afin de définir un état de pose de prothèse et rétractable, de manière coulissante vers la position médiale de l'élément tubulaire interne (150) afin de définir un état non gainé de prothèse ;
un fil-guide allongé (102) disposé, de manière coulissante, à l'intérieur de l'élément tubulaire interne (150) et s'étendant à partir de l'ensemble de poignée (170), à travers la patte ipsilatérale (126) de la prothèse (106) et à travers le corps tubulaire interne (124) de la prothèse (106) et s'étendant au-delà de l'extrémité ouverte du corps tubulaire principal (124) à l'état de pose de prothèse ; et
**caractérisé en ce que** le système de pose endovasculaire (100) comprend :
un ballonnet d'occlusion gonflable (200) disposé sur une partie du fil-guide allongé (102) s'étendant au-delà de l'extrémité ouverte du corps tubulaire principal (124) ;
dans lequel le fil-guide allongé (102) comprend une partie creuse (206) de sorte que le ballonnet d'occlusion gonflable (200) est en communication de fluide avec un matériau de gonflage de ballonnet ;
dans lequel le système de pose endovasculaire (100) a un diamètre externe global d'environ 3,3 mm (10 unités Charrière) à environ 4,7 mm (14 unités Charrière) ou inférieur à 3,3 mm (10 unités Charrière) ;
dans lequel le ballonnet d'occlusion gonflable (200) a une paroi ayant une épaisseur d'environ 0,01 mm (0,0005 pouce) à environ 0,08 mm (0,003 pouce) ; et
dans lequel le fil-guide allongé (102) a un diamètre externe d'environ 0,64 mm (0,025 pouce) à environ 1,1 mm (0,045 pouce).

2. Système de pose endovasculaire (100) selon la revendication 1, dans lequel ledit corps tubulaire principal (124) comprend un manchon gonflable (134) disposé à proximité de l'extrémité proximale ouverte.

3. Système de pose endovasculaire (100) selon l'une quelconque des revendications précédentes, dans lequel la partie du fil-guide allongé (102) sur laquelle le ballonnet d'occlusion (200) est disposé, présente une porosité (204) pour l'entrée et la sortie du matériau de gonflage de ballonnet.

4. Système de pose endovasculaire (100) selon la revendication 3, dans lequel la porosité (204) est une pluralité de trous (204) disposés à travers le fil-guide allongé (102).

5. Système de pose endovasculaire (100) selon l'une quelconque des revendications précédentes, dans lequel le matériau de gonflage de ballonnet comprend une solution saline et un matériau de contraste.

6. Système de pose endovasculaire (100) selon l'une quelconque des revendications précédentes, dans lequel le fil-guide allongé (102) comprend un matériau métallique.

7. Système de pose endovasculaire (100) selon l'une quelconque des revendications 1 à 5, dans lequel le fil-guide allongé (102) comprend un matériau polymère.

8. Système de pose endovasculaire (100) selon l'une quelconque des revendications précédentes, comprenant en outre un mandrin amovible (210) disposé à l'intérieur du fil-guide allongé (102) pour supporter le fil-guide allongé (102) avant le gonflage du ballonnet d'occlusion (200).

9. Système de pose endovasculaire (100) selon l'une quelconque des revendications précédentes, comprenant en outre un joint d'étanchéité (212) disposé sur une partie proximale du fil-guide allongé (102), telle qu'une partie disposée à l'intérieur de l'ensemble de poignée (170).

10. Système de pose endovasculaire (100) selon l'une quelconque des revendications précédentes, dans lequel lesdites pattes ipsilatérale et contralatérale (126, 128) comprennent des canaux gonflables (136).

11. Système de pose endovasculaire (100) selon l'une quelconque des revendications précédentes, comprenant en outre un premier marqueur radio-opaque (202) disposé sur une partie du fil-guide allongé (102) juste après le ballonnet d'occlusion (200) .

12. Système de pose endovasculaire (100) selon la revendication 11, comprenant en outre un second marqueur radio-opaque (202) disposé sur une partie du fil-guide allongé (102) juste avant le ballonnet d'occlusion (200).

13. Système de pose endovasculaire (100) selon l'une quelconque des revendications précédentes, comprenant en outre :
une seringue (250) ayant un matériau gonflable pour gonfler le ballonnet d'occlusion gonflable (200) ; et un raccord Luer (230) pour fournir la communication de fluide du matériau gonflable avec la partie de lumière creuse (206) du fil-guide allongé (102).

14. Système de pose endovasculaire (100) selon l'une quelconque des revendications précédentes, dans lequel le fil-guide (102) a un diamètre externe d'environ 0,89 mm (0,035 pouce) et un diamètre de lumière creuse d'environ 0,38 mm (0,015 pouce) à environ 0,76 mm (0,030 pouce).

15. Système de pose endovasculaire (100) selon l'une quelconque des revendications 1 à 6 et 8 à 12, dans lequel le fil-guide allongé (102) comprend un matériau polymère pour définir un fil-guide polymère (102), dans lequel le fil-guide polymère (102) comprend une bobine de renforcement (208) ou des fibres ou des particules de renforcement.
